# EUROPÄISCHE PATENTSCHRIFT

(11) **EP 4 035 422 B1**
(45) Veröffentlichungstag und Bekanntmachung des Hinweises auf die Patenterteilung: **28.01.2026**
(21) Anmeldenummer: 20830158.0
(22) Anmeldetag: 16.12.2020
(51) Int. Cl.: H04R 25/00, G08B 21/04, A61B 5/11, A61B 5/00, G08B 29/18

(54) **VERFAHREN ZUM BETREIBEN EINES HÖRSYSTEMS UND HÖRSYSTEM**
METHOD FOR OPERATING A HEARING SYSTEM AND HEARING SYSTEM
PROCÉDÉ DE FONCTIONNEMENT D'UN SYSTÈME AUDITIF ET SYSTÈME AUDITIF

(43) Veröffentlichungstag der Anmeldung: 03.08.2022
(73) Patentinhaber: Sivantos Pte. Ltd., Singapore 539775 (SG)
(72) Erfinder: HANNEMANN, Ronny, 91054 Buckenhof (DE); WURZBACHER, Tobias, 90768 Fürth (DE)
(74) Vertreter: FDST Patentanwälte
(86) Internationale Anmeldenummer: PCT/EP2020/086518
(87) Internationale Veröffentlichungsnummer: WO 2022/128082

(56) Entgegenhaltungen:
- WO-A1-2020/206155
- WO-A2-2020/124022
- US-A1- 2018 228 404
- US-A1- 2020 205 746

## Beschreibung

Die Erfindung betrifft ein Verfahren zum Betreiben eins Hörsystems, welches ein Hörgerät mit mindestens einem Mikrofon und mit einem Hörer sowie mit einem Bewegungssensor aufweist. Die Erfindung betrifft weiterhin ein Hörsystem, insbesondere eine Hörhilfevorrichtung, zur Durchführung des Verfahrens.

Hörhilfevorrichtungen sind tragbare Hörgeräte, die zur Versorgung von Schwerhörenden oder Hörgeschädigten dienen. Um den zahlreichen individuellen Bedürfnissen entgegenzukommen, werden unterschiedliche Bauformen von Hörhilfevorrichtungen wie Hinter-dem-Ohr-Hörgeräte (HdO) und Hörgeräte mit einem externen Hörer (RIC: receiver in the canal) sowie In-dem-Ohr-Hörgeräte (IdO), zum Beispiel auch Concha-Hörgeräte oder Kanal-Hörgeräte (ITE: In-The-Ear, CIC: Completely-In-Channel, IIC: Invisible-In-The-Channel), bereitgestellt. Die beispielhaft aufgeführten Hörgeräte werden am Außenohr oder im Gehörgang eines Hörhilfevorrichtungsnutzers getragen. Darüber hinaus stehen auf dem Markt aber auch Knochenleitungshörhilfen, implantierbare oder vibrotaktile Hörhilfen zur Verfügung. Dabei erfolgt die Stimulation des geschädigten Gehörs entweder mechanisch oder elektrisch.

Derartige Hörgeräte besitzen prinzipiell als wesentliche Komponenten einen Eingangswandler, einen Verstärker und einen Ausgangswandler. Der Eingangswandler ist in der Regel ein akusto-elektrischer Wandler, wie beispielsweise ein Mikrofon, und/oder ein elektromagnetischer Empfänger, zum Beispiel eine Induktionsspule oder eine (Radiofrequenz-, RF-)Antenne. Der Ausgangswandler ist meist als ein elektro-akustischer Wandler, zum Beispiel als ein Miniaturlautsprecher (Hörer), oder als ein elektromechanischer Wandler, wie beispielsweise ein Knochenleitungshörer, realisiert. Der Verstärker ist üblicherweise in eine Signalverarbeitungseinrichtung integriert. Die Energieversorgung erfolgt üblicherweise durch eine Batterie oder einen aufladbaren Akkumulator.

Bei einer sogenannten binauralen Hörhilfevorrichtung werden zwei derartige Hörgeräte von einem Benutzer getragen, wobei zwischen den Hörgeräten eine Kommunikationsverbindung besteht. Im Betrieb werden hierbei beispielsweise drahtlos Daten, gegebenenfalls auch große Datenmengen, zwischen dem Hörgerät am rechten und linken Ohr ausgetauscht. Die ausgetauschten Daten und Informationen ermöglichen eine besonders effektive Anpassung der Hörgeräte an eine jeweilige akustische Umgebungssituation. Insbesondere wird hierdurch ein besonders authentischer Raumklang für den Benutzer ermöglicht sowie das Sprachverständnis, auch in lauten Umgebungen, verbessert.

Mit fortschreitendem Alter besteht bei Menschen eine zunehmende Gefahr von Erkrankungen oder Komorbiditäten, insbesondere des autonomen Nervensystems (ANS), wie beispielsweise einem Hörverlust, Demenz, oder Parkinson-Krankheit. Insbesondere treten häufig mehrere solche Erkrankungen auf, so dass Personen mit Demenz oder Parkinson regelmäßig auch eine Hörstörung oder einen Hörverlust aufweisen.

Viele dieser Krankheiten und Störungen führen auch zu einem höheren Sturzrisiko und schließlich zu Sturzereignissen der betroffenen Person, welche zu schweren Verletzungen führen können. Hierbei sind auch psychologische Folgen oder Auswirkungen von Sturzereignissen zu berücksichtigen, wie beispielsweise Angstzustände nach einem Sturz. Ein Sturz kann somit zu einer verminderten Lebensqualität und erhöhten Kosten im Gesundheitswesen führen. Die meisten Stürze könnten verhindert werden, wenn es eine Früherkennung des Sturzrisikos gäbe, wodurch eine hohe Lebensqualität erhalten und die Kosten für die Gesundheitssysteme gesenkt werden könnten.

Zur Überwachung verschiedener Arten von ANS-Störungen und zur Beurteilung des damit verbundenen Sturzrisikos ist es beispielsweise denkbar Bewegungssensoren oder Bewegungs-Erfassungs-Vorrichtungen (Motion-Capture) einzusetzen, welche die Bewegungen oder eine Bewegungsstörung der betroffenen Person erfassen und auswerten. Hierbei ist jedoch in der Regel eine zusätzliche Hardware und/oder ein Experte notwendig, welche(r) eine zuverlässige Platzierung der Sensoren gewährleistet.

Derartige klinischen Bewertungen oder stationären Motion-Capture-Systeme weisen im klinischen Kontext jedoch eine Anzahl von Nachteilen auf.

So weisen die im klinischen Kontext häufig verwendeten Methoden zur Sturzrisikobewertung (engl.: Fall Risk Assessment, FRA) eine schlechte bis mäßige diagnostische Genauigkeit oder Sturzvorhersage bei gesunden hochfunktionellen älteren Erwachsenen auf, und sind von der Expertise des Beobachters abhängig.

Des Weiteren spiegeln aufgrund des Beobachtereffekts bei kontrollierten Gang- und Gleichgewichtstests im Labor überwachte FRA-Messungen möglicherweise kein naturalistisches und multitaskingfähiges Verhalten der Person wider.

Eine (visuelle) 3-dimensionale Bewegungserfassung (Motion-Capturing) und instrumentierte Gehwege sind ressourcenintensiv und an spezialisierte Kliniken/Orte gebunden und ermöglichen lediglich Momentaufnahmen während vorgegebenen funktionellen Aufgaben.

Ebenso denkbar ist beispielsweise der Einsatz von Umgebungssensoren, insbesondere von Kameras, zur Überwachung und Erfassung von Personenbewegungen. Derartige Umgebungssensoren weisen jedoch Einschränkungen aufgrund von visuellen Okklusionen und der Verfolgung derselben Person in Räumen mit mehreren Personen mit ähnlichen Körpermerkmalen auf.

Für die Diagnose und Überwachung von ANS-Erkrankungen gibt es bisher kein einfach zu handhabendes, objektives und zuverlässiges Diagnose-/Überwachungssystem, das sich leicht selbst verwalten lässt. Selbst wenn ein gut ausgebildeter / erfahrener medizinischer Experte den Zustand und das Fortschreiten von ANS-Erkrankungen in einem Labor beurteilen könnte, fehlt es an Lösungen, die im täglichen Lebensumfeld eines Patienten anwendbar sind. Bis heute benötigen ältere Personen, die an Hörschäden und anderen ANS-Störungen leiden, viele unabhängige Lösungen für jede Störung einzeln.

Aus der WO 2020/206155 A1 ist ein Überwachungssystem mit einem ersten und einem zweiten Sensor bekannt. Die Sensoren sind jeweils geeignet, ein Merkmal eines Subjekts des Systems zu erfassen und Daten zu erzeugen, die für das Merkmal des Subjekts repräsentativ sind. Eine Steuereinheit, die mit den Sensoren gekoppelt ist, ist so eingerichtet, dass sie Daten von dem ersten und dem zweiten Sensor empfängt, die für ein erstes und ein zweites Merkmal des Subjekts repräsentativ sind, und dass sie auf der Grundlage der von dem ersten und dem zweiten Sensor empfangenen Daten Statistiken für einen ersten und einen zweiten Zustandsunterzustand des Subjekts über eine Überwachungsperiode bestimmt. Die Steuereinheit ist ferner dazu eingerichtet, die Statistiken der ersten und zweiten Zustandsteilzustände zu vergleichen, den ersten Zustandsteilzustand zu bestätigen, wenn er dem zweiten Zustandsteilzustand im Wesentlichen ähnlich ist, und die Statistiken eines Gesamtzustands des Subjekts auf der Grundlage des bestätigten ersten Zustandsteilzustands zu bestimmen.

In der US 2020/205746 A1 wird ein System zur Vorhersage von Sturzereignissen offenbart. Das System umfasst eine am Körper getragene Vorrichtung und ein Steuergerät, das betriebsmäßig mit der am Körper getragenen Vorrichtung verbunden ist. Die Steuereinheit ist so eingerichtet, dass sie physiologische Daten empfängt, die eine physiologische Eigenschaft eines Trägers der am Körper getragenen Vorrichtung über einen Überwachungszeitraum darstellen; kontextbezogene Daten empfängt, die Kontextinformationen des Trägers über den Überwachungszeitraum darstellen; und einen oder mehrere zukünftige physiologische Zustände oder kontextbezogene Zustände zumindest teilweise auf der Grundlage der physiologischen Daten oder der kontextbezogenen Daten bestimmt. Die Steuereinheit ist ferner dafür ausgelegt, für einen zukünftigen Zeitpunkt zu bestimmen, ob eine Sturzbedingung erfüllt ist, basierend auf dem einen oder den mehreren zukünftigen physiologischen Zuständen oder Kontextzuständen, und eine Sturzpräventionsausgabe zu erzeugen, die auf die Erfüllung der Sturzbedingung reagiert.

Die US 2018/228404 A1 offenbart ein System zur Sturzvorhersage. Das System umfasst ein Hörgerät für einen Träger, einen Sensor, der betriebsmäßig mit dem Hörgerät verbunden ist und dazu ausgelegt ist, ein Merkmal des Trägers zu erfassen und ein Sensorsignal auf der Grundlage des Merkmals zu erzeugen, und eine Steuereinheit, die betriebsmäßig mit dem Hörgerät verbunden ist. Die Steuereinheit ist dafür ausgelegt, einen Sturzrisikowert auf der Grundlage des Sensorsignals zu bestimmen, den Sturzrisikowert mit einem Sturzrisikogrenzwert zu vergleichen und eine Sturzpräventionsausgabe zu erzeugen, wenn der Sturzrisikowert den Sturzrisikogrenzwert überschreitet.

Der Erfindung liegt die Aufgabe zugrunde, ein besonders geeignetes Verfahren zum Betrieb eines Hörsystems anzugeben. Insbesondere soll eine Früherkennung eines Sturzrisikos des Hörsystemnutzers realisiert werden. Vorzugsweise soll eine Behandlung von Hörstörungen mit der Überwachung anderer ANS-Erkrankungen verbunden werden. Der Erfindung liegt weiterhin die Aufgabe zugrunde, ein besonders geeignetes Hörsystem zur Durchführung des Verfahrens anzugeben.

Hinsichtlich des Verfahrens wird die Aufgabe mit den Merkmalen des Anspruchs 1 und hinsichtlich des Hörsystems mit den Merkmalen des Anspruchs 10 erfindungsgemäß gelöst. Vorteilhafte Ausgestaltungen und Weiterbildungen sind Gegenstand der Unteransprüche.

Die im Hinblick auf das Verfahren angeführten Vorteile und Ausgestaltungen sind sinngemäß auch auf das Hörsystem übertragbar und umgekehrt. Sofern nachfolgend Verfahrensschritte beschrieben werden, ergeben sich vorteilhafte Ausgestaltungen für das Hörsystem insbesondere dadurch, dass dieses ausgebildet ist, einen oder mehrere dieser Verfahrensschritte auszuführen.

Das erfindungsgemäße Verfahren ist zum Betreiben eines Hörsystems, insbesondere einer Hörhilfevorrichtung, vorgesehen sowie dafür geeignet und ausgestaltet. Das Hörsystem weist ein Hörgerät auf.

Das Hörgerät dient insbesondere der Versorgung eines hörgeschädigten Nutzers (Hörsystemnutzer). Das Hörgerät ist hierbei ausgebildet, Schallsignale aus der Umgebung aufzunehmen und an einen Nutzer des Hörgeräts auszugeben. Hierzu weist das Hörgerät zumindest einen akusto-elektrischen Eingangswandler, insbesondere ein Mikrofon, auf sowie zumindest einen elektro-akustischen Ausgangswandler, beispielsweise einen Hörer. Der Eingangswandler nimmt im Betrieb des Hörgeräts Schallsignale (Geräusche, Töne, Sprache, etc.) aus der Umgebung auf, und wandelt diese in ein elektrisches Eingangssignal (Akustikdaten) um. Aus dem elektrischen Eingangssignal wird ein elektrisches Ausgangssignal erzeugt, indem das Eingangssignal in einer Signalverarbeitung modifiziert wird. Die Signalverarbeitung ist beispielsweise ein Teil des Hörgeräts. Der Eingangswandler und der Ausgangswandler sowie gegebenenfalls auch die Signalverarbeitung sind insbesondere in einem Gehäuse des Hörgeräts untergebracht. Das Gehäuse ist derart ausgebildet, dass dieses vom Nutzer am Kopf und in der Nähe des Ohrs getragen werden kann, z.B. im Ohr, am Ohr oder hinter dem Ohr. Vorzugsweise ist das Hörgerät als BTE-Hörgerät, ITO-Hörgerät oder RIC-Hörgerät ausgebildet.

Das Hörgerät weist weiterhin einen Bewegungssensor zur Erfassung von Bewegungen der Hörsystemnutzers auf. Der Bewegungssensor ist dazu vorgesehen und eingerichtet dreidimensionale (Körper-)Bewegungen oder Bewegungsereignisse, insbesondere translatorische und/oder rotatorische Bewegungen, zu erfassen. Der Bewegungssensor ist hierbei beispielsweise als ein Beschleunigungsmesser und/oder als ein Gyroskop, also als ein gyroskopischer (Lage-)Sensor, ausgebildet. Der Bewegungssensor kann alternativ auch ein Puls-, Blutdruck- oder Lichtsensor sein. Ebenso möglich ist eine Kombination aus Beschleunigungsmesser und/oder Gyroskop und/oder Pulssensor und/oder Blutdrucksensor und/oder Lichtsensor. Der Bewegungssensor ist hierbei vorzugsweise in dem Hörgerät, insbesondere in dessen Gehäuse, integriert.

Die Konjunktion "und/oder" ist hier und im Folgenden derart zu verstehen, dass die mittels dieser Konjunktion verknüpften Merkmale sowohl gemeinsam als auch als Alternativen zueinander ausgebildet sein können.

Im Anwendungsfall ist das Hörgerät am Kopf und in der Nähe des Ohrs des Hörsystemnutzers getragen, so dass mittels des Bewegungssensors insbesondere Kopfbewegungen der Hörsystemnutzers erfassbar sind. Insbesondere sind somit zumindest mittelbar auch Geh- oder Gangbewegungen, also Körperbewegungen aufgrund eines Gehens oder Laufens, über den Bewegungssensor des Hörgeräts erfassbar.

Verfahrensgemäß wird während des Betriebs des Hörsystems eine Bewegung des Hörsystemnutzers als Bewegungsdaten des Bewegungssensors erfasst. Unter Bewegungsdaten sind hierbei insbesondere richtungsabhängige Beschleunigungen oder Drehungen zu verstehen, insbesondere ein daraus abgeleitetes Bewegungsmuster. Die Bewegungsdaten sind somit ein Maß für die vom Hörsystemnutzers ausgeführten Körperbewegungen, insbesondere für Gehbewegungen.

Erfindungsgemäß wird anhand der erfassten Bewegungsdaten eine Wahrscheinlichkeit für ein zukünftiges Fall- oder Sturzereignis des Hörsystemnutzers bestimmt. Mit anderen Worten werden die Bewegungsdaten hinsichtlich eines Sturzrisikos ausgewertet, insbesondere wird das Sturzrisiko also eine Fall- oder Sturz-wahrscheinlichkeit beziehungsweise ein Maß hierfür anhand der Bewegungsdaten prognostiziert oder (ab-)geschätzt. Unter einem Fall- oder Sturzereignis des Hörsystemnutzers ist hierbei ein Fall oder ein Sturz, also ein Hinfallen oder Umfallen oder Stürzen, der das Hörgerät tragenden Person zu verstehen.

Unter "Prognose" oder "prognostizieren" ist hier und im Folgenden insbesondere eine vorausschauende Abschätzung, also eine Prädiktion, zu verstehen, bei welcher anhand aktueller und/oder vergangener Bewegungsdaten - gegebenenfalls unter Hinzunahme von mathematischen oder physikalischen Modellen - ein zukünftiges oder zu erwartendes Sturzrisiko berechnet oder prädiziert wird, welches mit einer hinreichenden Wahrscheinlichkeit auftritt. Welche Wahrscheinlichkeit hierbei als hinreichend gilt und wie groß die Wahrscheinlichkeit konkret ist, ist dabei zunächst nebensächlich. Dies lässt sich beispielsweise aus vergangenen Bewegungsdaten oder aus entsprechenden Versuchen oder Erprobungen ermitteln. Für unterschiedliche Personen ergeben sich aufgrund von unterschiedlichen Alter, Gesundheitszustand oder Körperabmessungen unter Umständen unterschiedliche prognostizierte Sturzrisiken.

Unter "Schätzung" oder "schätzen" ist hier und im Folgenden eine genäherte Bestimmung der Sturzwahrscheinlichkeit durch Auswertung der Bewegungsdaten, beispielsweise durch Mustererkennung, vorcharakterisierten Messungen, hinterlegten Tabellen oder Kennlinien, oder mittels statistisch-mathematischer Methoden, zu verstehen.

Erfindungsgemäß wird ein wahrnehmbares Signal als Sturz- oder Fallwarnung (Warnsignal) erzeugt, wenn die Wahrscheinlichkeit oder das Sturzrisiko einen hinterlegten Schwellwert erreicht oder überschreitet. Mit anderen Worten wird eine Warnung für den Hörsystemnutzer erzeugt, wenn ein hinreichend hohes Sturzrisiko vorliegt. Welche Wahrscheinlichkeit oder Sturzrisiko hierbei als hinreichend gilt und wie groß die Wahrscheinlichkeit konkret ist, ist dabei zunächst nebensächlich. Dies lässt sich beispielsweise aus vergangenen Bewegungsdaten oder aus entsprechenden Versuchen oder Erprobungen ermitteln. Für unterschiedliche Personen ergeben sich aufgrund von unterschiedlichen Alter, Gesundheitszustand oder Körperabmessungen unter Umständen unterschiedliche Schwellwerte.

Das Warnsignal ist beispielsweise ein optisches und/oder akustisches Signal, beispielsweise eine leuchtende oder blinkende LED oder ein Warnton oder Warngeräusch, welcher/s von dem Hörgerät beziehungsweise dem Ausgangswandler erzeugt wird. Ebenso denkbar ist beispielsweise ein haptisches Vibrationssignal, welches zum Beispiel von einem signaltechnisch mit dem Hörgerät gekoppelten Zusatzgerät, insbesondere einem Smartphone, erzeugt wird. Zusätzlich oder alternativ ist es weiterhin denkbar, dass das Warnsignal über eine mit dem Hörgerät gekoppelte Anwendung, beispielsweise eine Smartphone-App, an einen Arzt oder an ein Pflegepersonal ausgegeben wird. Das Warnsignal kann auch eine Kombination aus akustischen, optischen und haptischen Signalen sein.

Durch das Warnsignal wird der Benutzer des Hörsystems über die Gefahr oder das Risiko eines Sturzes während des Tragens informiert. Dem Hörsystemnutzer wird also signalisiert, die Gefahr eines sich anbahnenden Sturzereignisses besteht. Somit wird die Gefahr eines Sturzes oder eines Sturzereignisses vorteilhaft reduziert. Dadurch ist ein besonders geeignetes Verfahren zum Betrieb eines Hörsystems realisiert. Durch das erfindungsgemäße Verfahren ist somit ein besonders vorteilhafter Sturz- oder Fallschutz bereitgestellt.

Der Bewegungssensor im Hörgerät, welches zur Kompensation einer Hörschädigung getragen wird, wird somit erfindungsgemäß zur Überwachung der Bewegungsmuster des Hörgeräteträgers genutzt, und hieraus ein Sturzrisiko bestimmt. Ein Kerngedanke der Erfindung besteht somit insbesondere darin, die Bewegungen des Hörsystemnutzers (bspw. Gang, Drehung, usw.) mittels des Hörgeräts zu erfassen, sowie die erfassten Bewegungen zu analysieren und hinsichtlich eines Sturzrisikos auszuwerten.

Die analysierten oder ausgewerteten Bewegungsdaten ermöglichen beispielsweise eine Charakterisierung einer fortschreitenden ANS-Störung. Grundsätzlich ist durch die erfindungsgemäße Bestimmung eines Sturzrisikos auch eine Früherkennung oder Diagnose von ANS-Störungen möglich. Dies bedeutet, dass das Verfahren im Rahmen einer Diagnose oder einer Behandlung von ANS-Störungen eingesetzt werden kann. Die Diagnose oder Behandlung von ANS-Störungen ist jedoch nicht Teil der beanspruchten Erfindung.

Das Hörgerät weist beispielsweise einen Speicher auf und ist ausgebildet, innerhalb eines Zeitfensters die Bewegungsdaten, welche vom Bewegungssensor aufgenommen werden, in dem Speicher zu speichern und zu einem späteren Zeitpunkt hinsichtlich des Sturzrisikos auszuwerten (Monitoring). Das Hörgerät ist also beispielsweise ausgebildet, zeitlich begrenzte Ausschnitte des Bewegungssensorsignals im Speicher zu speichern und insbesondere zur Bestimmung der Sturzwahrscheinlichkeit auszugeben. Das Hörgerät weist also eine Aufnahmefunktion (Recording) für die Bewegungsdaten auf. Zweckmäßigerweise sind die aufgenommenen Bewegungsdaten aus dem Speicher über eine Schnittstelle auch an eine externe Datenquelle übertragbar. Das Zeitfenster weist beispielsweise eine Länge von wenigstens 1 min (Minute) bis hin zu 1 d (Tag) auf, besonders bevorzugt ist eine Länge im Bereich von 1 h (Stunde) bis 12 h. Geeignet sind aber auch andere Längen. Insbesondere wird ein Zeitfenster verwendet, welche der Tragedauer des Hörgeräts entspricht. Dies bedeutet, dass die Aufnahme beim Aufsetzen des Hörgeräts gestartet und beim Abnehmen des Hörgeräts beendet wird.

Ebenso denkbar ist beispielsweise eine eventbasierte Aufnahme und Auswertung, bei welcher ein (Betriebs-)Algorithmus beispielsweise erfasst, ob und wann der Hörsystemnutzer läuft. Wird kein Laufen erfasst, so erfolgt vorzugsweise ein simpler und stromsparender Modus, wobei im Falle eines Laufens die Abtastrate erhöht und weitere Berechnungen, Analyse und Speicherungen basierend auf den Sensorrohdaten erfolgen. Dies bedeutet, dass das Zeitfenster dynamisch anhand von erfassten Körperbewegungen eingestellt/ausgelöst werden kann. Dadurch ist ein längeres Beobachtungs-/Auswertungsfenster, beispielsweise über einen Tag hinaus, ermöglicht.

Weiterhin ist es beispielsweise möglich ein periodisches oder regelmäßiges Zeitfenster zur Überwachung und Auswertung vorzusehen, zum Beispiel wird lediglich maximal eine Periode pro Tag oder Woche aufgenommen, wobei ein Algorithmus beispielsweise lediglich einmal pro Woche ein Zeitfenster von beispielsweise 10 min erfasst.

Zur Bewegungsanalyse und Risikoprognose werden beispielsweise Makro- und Mikroereignisse genutzt, welche den Gang des Hörsystemnutzers charakterisieren, und darüber hinaus in Drehungen und Übergänge zwischen den Bewegungen kategorisiert werden.

Unter einem "Mikroereignis" ist hier und im Folgenden insbesondere eine Charakterisierung von (Bewegungs-)Merkmalen einer Bewegung, also ein Bewegungsablauf oder ein Bewegungsmuster, zu verstehen. Unter einem "Makroereignis" sind hier und im Folgenden insbesondere mehrere Bewegungen oder Bewegungsgruppen innerhalb eines Zeitintervalls, beispielsweise an einem Tag, also eine Bewegungssequenz oder Bewegungsabfolge, zu verstehen.

Unter einem Makroereignis ist hierbei beispielsweise die Anzahl der Schritte pro Zeitintervall, das heißt die Anzahl der Schritte pro Tag oder die Anzahl pro Aktion im Falle eines vordefinierten Tests zur Charakterisierung der Intensität (Kadenz, Schrittfrequenz), zu verstehen.

Unter einem Makroereignis ist weiterhin ein absoluter und relativer Zeitaufwand pro klassifizierter Aktivität, das heißt zum Beispiel pro Tag aufgewendete Gehzeit, ein Prozentsatz von Gehen und Nicht-Gehen, eine absolute Dauer des längsten Spaziergangs oder dergleichen zu verstehen. Dies beruht auf der Erkenntnis, dass eine Gesamtgehzeit für potentielle Sturzgefährdeten im Vergleich zu Nicht-Sturzgefährdeten häufig reduziert ist. Weiterhin ist eine Gesamtzeit, welche für so genannte sitzende Verhalten aufgewendet wird, für sturzgefährdete Personen üblicherweise länger.

Makroereignisse sind weiterhin bewegungsarme Verhalten, wie Stehen, Sitzen, oder Liegen. Diese können beispielsweise hinsichtlich einer Gesamtzeit, welche zum Beispiel pro Tag jeweils für diese Verhaltensweisen aufgewendet wird. Ebenso kann eine Dauer für das Einnehmen dieser Verhaltensweisen, also eine Dauer für ein Hinsetzen oder Aufstehen, zur Charakterisierung dieser Verhalten verwendet werden. Hierbei kann beispielsweise ein Mittelwert oder Maximum oder Perzentil, insbesondere ein 90%-Perzentil, von mehreren Aufsteh- oder Hinsetzbewegungen zur Qualifizierung bestimmt werden. Weiterhin kann eine Variabilität der Zeit pro Tag und Dauer des sitzenden Verhaltens berücksichtigt werden.

Unter Makroereignissen ist beispielsweise auch eine Varianz der Bewegungen von Tag/Aktion zu Tag/Aktion über einen Zeitraum zu verstehen, um die Qualität der Bewegungen zu charakterisieren. Zum Beispiel ist ein Fortschreiten von Demenz durch eine Zunahme der Tag-Tag-Variabilität der Gangmuster gekennzeichnet, woraus ein Sturzrisiko ableitbar ist.

Ein Mikroereignis ist hierbei beispielsweise das Tempo einer Bewegung, also zum Beispiel eine Schrittgeschwindigkeit oder "Ganggeschwindigkeit", oder eine Schritt-Schwungzeit-Variabilität. Potenzielle Stürzende gehen langsamer wobei eine Veränderungen in der Ganggeschwindigkeit wie vorstehend erläutert ein Fortschreiten bei Demenz charakterisieren kann. Die Ganggeschwindigkeit kann hierbei in vordefinierten (Zeit-)Intervallen analysiert werden, beispielsweise kleiner als 10 s (Sekunden), 10 s bis 60 s, 60 s bis 120 s, länger als 120 s. Eine grobe Schätzung des Tempos ist hierbei ausreichend, eine genaue Messung oder Bestimmung der Geschwindigkeit in Meter pro Sekunde (m/s) ist nicht erforderlich. Zur Bestimmung des Tempos oder der Geschwindigkeit können die Bewegungsdaten als Rohwerte oder in Form von Effektivwerten analysiert werden.

Als Mikroereignis ist beispielsweise auch eine Schrittbewegung (Pace), also zum Beispiel die Schrittlänge, zu verstehen. Die Schrittlänge kann mit fortschreitender Demenz abnehmen, so dass sich aus einer Bestimmung oder Schätzung der Schrittlänge ein Sturzrisiko prognostizieren lässt. Eine Schrittlängenschätzung für hüft- oder brustgetragene Bewegungssensoren ist beispielsweise in der Veröffentlichung Analog Devices Application Note, AN-602 unter dem Titel "Using the ADXL202 in Pedometer and Personal Navigation Applications" von H. Weinberg beschrieben.

Als Mikroereignis wird beispielsweise auch ein Rhythmus der Bewegung, beispielsweise Schritt, Schwung und Standzeit, oder eine Asymmetrie der Bewegung, also beispielsweise eine Schritt-, Schwung- und Standzeitvariabilität, verstanden. Bei einer Asymmetrie können beispielsweise die Schritt-, Schwung- und Standzeitvariabilität als Parameter ein als "Gangglätte" (engl.: gait smoothness) bezeichnetes Bewegungsmerkmal charakterisieren, also eine "Schritt-zu-Schritt"-Symmetrie, eine Gehstabilität, oder ein harmonisches Verhältnis (engl.: harmonic ratio, HR).

Wenn das Verfahren beispielsweise im Rahmen einer Diagnose oder Behandlung von ANS-Störungen eingesetzt wird, kann parallel zum Verfahren eine Herzfrequenz des Hörsystemnutzers getrennt analysiert werden, beispielsweise antero-posterior (AP), medio-lateral (ML) und superior-inferior (V) entsprechend der Laufrichtung. Beispielsweise ist die Herzfrequenz ist in frühen Stadien der Demenz reduziert, wobei Veränderungen bei Parkinson signifikant mit einem kognitiven Status, also insbesondere der Herzfrequenz in AP-Richtung, des Patienten korreliert sind.

Als Mikroereignis kann auch eine Haltungskontrolle zur Überwachung einer Körperhaltung des Hörsystemnutzers dienen. Beispielsweise wird eine Schritt- oder Schrittlängenasymmetrie zur Überprüfung von Haltungsmerkmalen und/oder einer (Haltungs-/Bewegungs-)Stabilität des Hörsystemnutzers genutzt.

Ein Mikroereignis ist beispielsweise weiterhin eine Variabilität der Schrittlänge und/oder der Schrittgeschwindigkeit innerhalb und zwischen verschiedenen Bewegungsmustern, zum Beispiel bei einem Spaziergang.

Weiterhin ist eine Variabilität einer Stand- und/oder Schrittzeit als Mikroereignis anzusehen, welche auch zwischen und innerhalb vergleichbarer Aktionen oder Bewegungen erfolgen kann.

Ein Mikroereignis kann auch eine andere Variabilität des Gangmusters sein, welche durch spektro-temporale (Leistungs-)Verteilungsmuster in verschiedenen Frequenzbändern für jede Richtung (das heißt antero-posterior (AP) entsprechend der Laufrichtung, medio-lateral (ML) und superior-inferior (V)) oder durch Autokorrelationen in dominanten Dimensionen charakterisiert sind.

Die temporäre Veränderungen in benutzertypischen Varianzen der oben erwähnten "Merkmale" oder Bewegungen (Variabilität der Schrittlänge und/oder der Schrittgeschwindigkeit, Variabilität einer Stand- und/oder Schrittzeit, Variabilität des Gangmusters) können kompensatorische Gleichgewichtsreaktionen des Hörsystemnutzers charakterisieren, welche notwendig sind, um Stürze zu vermeiden.

Derartige kompensatorische Gleichgewichtsreaktionen können beispielsweise als "Fehltritt"-Stolpern, Ausrutschen, oder Stolpern charakterisiert werden, wobei sich beispielsweise bei einer Zunahme der Häufigkeit derartiger kompensatorische Gleichgewichtsreaktionen ein höheres Sturzrisiko ergibt.

Bei der Bewegungsanalyse werden auch Dreh- oder Rotationsverhalten des Hörsystemnutzers ausgewertet. Personen mit einem erhöhten Sturzrisiko weisen bei Drehbewegungen in der Regel eine längere Drehzeitdauer auf, weiterhin führen sie Drehbewegungen seltener aus und zeigen bei gleichen Bewegungen inkonsistente Drehwinkel mit großer Varianz. Ein erhöhtes Sturzrisiko kann sich auch in einer reduzierten Konsistenz sowohl in der Winkelgröße des Drehens/der Drehbewegung als auch im Zeitpunkt eines großen Drehens über Tage hinweg abzeichnen.

Zur Analyse oder Auswertung des Drehverhaltens werden bei den Bewegungsdaten beispielsweise folgende Merkmale extrahiert und/oder analysiert: die Drehdauer, Drehgeschwindigkeitsspitze, eine Schrittanzahl während des Drehens, ein Drehwinkel in Kategorien (Viertel-, Halb-, Volldrehung; oder vergleichbare Kategorien von Drehwinkeln), eine Anzahl von Drehbewegungen pro vorstehender Kategorie pro Tag, eine Varianz der Anzahl, des Winkels und/oder der Geschwindigkeit der Drehungen über aufeinanderfolgende Tage. Bei binaural genutzten Hörgeräten können die aus den Bewegungsdaten extrahierten Merkmale der Einzelgeräte miteinander kombiniert werden, um das Drehen besser zu charakterisieren.

Bei der Bewegungsanalyse werden zur Bestimmung des Sturzrisikos oder der Sturzwahrscheinlichkeit vorzugsweise auch Übergänge zwischen Bewegungen und/oder Kombination von Bewegungen berücksichtigt. Beispielsweise wird eine Gesamtverteilung von verschiedenen Bewegungsaktivitäten, also eine Verteilung der Makroereignisse, analysiert. Ebenso denkbar ist eine Analyse der Varianz eines Aktivitätsmusters über die Zeit. Weiterhin wird beispielsweise eine Dauer von Übergängen, also ein längeres Sitzen-zu-Sitzen, oder Übergänge vom Gehen zum Sitzen erfasst, wobei Änderungen der Übergänge oder Dauer der Übergänge auf ein erhöhtes Sturzrisiko hinweisen können. Des Weiteren ist auch eine Analyse eines Zeitintervalls zwischen zwei oder mehreren Bewegungsereignissen, beispielsweise bei einem Treppensteigen, denkbar. Die Bewegungsdaten können auch hinsichtlich der Ausrichtung oder Orientierung des Bewegungssensors vor und nach einem Bewegungsereignis ausgewertet werden.

Die Bewegungsdaten können beispielsweise in diskreten Zeitintervallen erfasst und ausgewertet werden. Vorzugsweise erfolgt jedoch während des Betriebs des Hörsystems, insbesondere während des Tragens des Hörgeräts oder während des Zeitfensters, eine im Wesentlichen kontinuierliche oder unterbrechungsfreie Erfassung und Analyse der Bewegungsdaten. Im Wesentlichen kontinuierlich oder unterbrechungsfrei bedeutet in diesem Zusammenhang insbesondere quasikontinuierlich, also dass beispielsweise bei einer digitalen Erfassung und Auswertung die Messfrequenz/-geschwindigkeit des Bewegungssensors höher ist, insbesondere mindestens doppelt so hoch, wie die Frequenz- oder Geschwindigkeit einer zu erfassenden Körperbewegung.

Eine (quasi-)kontinuierliche Bewegungsüberwachung/-analyse und die Bestimmung des Sturzrisikos ermöglichen eine Analyse oder Diagnostik von ANS-Störungen, welche selbst von einer nicht spezialisierten Person (z.B. Krankenschwester, Physiotherapeut, Arzt; nicht nur von medizinischen Experten) in einem klinisch/ambulanten Umfeld und unter sehr ökologischen Bedingungen in relativ kurzer Zeit durchgeführt werden kann. Sie ermöglicht es dem medizinischen Fachpersonal, individuelle Ansätze für Rehabilitationsstrategien zu entwickeln, welche eine sicherere Navigation und eine Verringerung der Stürze und der damit verbundenen Kosten gewährleisten.

Eine kontinuierliche Erfassung und Auswertung der Bewegungsdaten ermöglichen weiterhin eine zuverlässige Überwachung, wenn sich die Bewegungsmuster im Laufe der Zeit ändern (verbessern/verschlechtern). Dadurch ist es möglich dem Hörsystemnutzer ein direktes und angemessenes Feedback zu geben, so dass dieser rechtzeitig reagieren kann. Somit ist nicht unbedingt ein Arzttermin zur Überwachung oder Überprüfung von Veränderungen der Bewegungsmuster notwendig, wodurch geringere Kosten im Gesundheitssystem entstehen.

Eine (kontinuierliche) Beurteilung unter Verwendung von Bewegungssensor-Informationen in Hörgeräten weist im Vergleich zu anderen Methoden zur Beurteilung von Gang/Drehmustern eine verbesserte diagnostische Genauigkeit des natürlichen oder gestörten Verhaltens auf.

Darüber hinaus ist das erfindungsgemäße Verfahren vergleichsweise wirtschaftlich, da lediglich tragbare Sensoren in einem Gerät verwendet werden, welches ohnehin getragen wird, um den Hörverlust zu kompensieren. Durch das erfindungsgemäße Verfahren ist somit eine vorteilhafte Funktionserweiterung des Hörgeräts zusätzlich zur Versorgung eines hörgeschädigten Nutzers realisiert.

Im Vergleich zu Umgebungssensoren, welche in Gebäuden installiert sind, wie zum Beispiel Kameras, weisen Informationen oder Bewegungsdaten von tragbaren Hörgerätesensoren einen größeren Nutzen über den Wohnraum hinaus auf, insbesondere ist es nicht notwendig, dass sich der Benutzer in einer überwachten Umgebung aufhält, da er das Sensorsystem mit sich herumführt.

Durch das erfindungsgemäße Verfahren ist es weiterhin nicht notwendig, dass der Hörsystemnutzer bei einer ANS-Störung ein zusätzliches Gerät mit sich führen muss. Mit anderen Worten gibt keine zusätzliche Belastung durch eine Verwendung eines anderen Geräts über das Hörgerät hinaus. Dies kann somit auch Überzeugung von lediglich leicht hörgeschädigten Personen beitragen, ein Hörgerät zur Verbesserung der Lebensqualität und zur Verhinderung von Verletzungen und/oder zur Überwachung von Sturzrisiken zu verwenden.

Eine Hörgeräte-gestützte Diagnostik mittels des erfindungsgemäßen Verfahrens könnte auch der Beurteilung von Störungen nach klinischen Protokollen zugutekommen, bei welchen Zeit und Muster für dedizierte Aktionen (für diagnostische Zwecke) analysiert werden. Derartige Protokolle umfassen beispielsweise Hinsetzen, Aufstehen (Stuhl-Steh-Test), Stehenbleiben mit offenen und geschlossenen Augen, oder strukturierte Testverfahren wie beim sogenannten Tinetti-Test (siehe beispielsweise Tinetti ME, Performance-oriented assessment of mobility problems in elderly patients, J Am Geriatr Soc 1986;34:119-126).

In einer vorteilhaften Ausführung des Verfahrens werden in einem Speicher des Hörsystems persönliche Gesundheitsdaten des Hörsystemnutzers hinterlegt, wobei die Bestimmung der Sturzwahrscheinlichkeit anhand der hinterlegten Gesundheitsdaten erfolgt. Dies bedeutet, dass bei der Auswertung oder Analyse der Bewegungsdaten zur Bestimmung des Sturzrisikos zusätzlich die hinterlegten Gesundheitsdaten berücksichtigt werden. Zusätzlich oder alternativ ist es beispielsweise ebenso denkbar, dass der Schwellwert anhand der Gesundheitsdaten variiert wird.

Diese Ausführung des Verfahrens geht von der Erkenntnis aus, dass altersbedingte Störungen oder ANS-Störungen mehrere Hauptmerkmale des Gangbildes verändern. Dadurch ist eine besonders zuverlässige und sichere Abschätzung oder Bestimmung des Sturzrisikos ermöglicht, so dass eine frühzeitige und rechtzeitige Warnung des Hörsystemnutzers gewährleistet ist.

Mit anderen Worten werden die aufgezeichneten Bewegungsdaten zusammen mit anderen (verfügbaren) Informationen über den Benutzer (d.h. Kognition, Gehör, Umgebung, Herz-Kreislauf-Status) analysiert, um eine benutzerspezifische Charakterisierung zu ermöglichen. Diese Charakterisierung wird für die Vorhersage von Veränderungen des Sturzrisikos im Zusammenhang mit bestimmten Störungen, aber auch für die Überwachung des Fortschreitens dieser Störungen verwendet.

Unter Gesundheitsdaten sind hierbei personenspezifische Informationen des Hörsystemnutzers, insbesondere physiologische und medizinische/gesundheitliche Daten des Hörsystemnutzers, wie beispielsweise Alter, Körpergröße, Bein-/Schrittlänge, Körpergewicht, Herz-Kreislauf-Status, kognitiver Status, Hörvermögen, Aktivität der eigenen Stimme, Tinnitus, zu verstehen. Dadurch ist eine zuverlässige Bestimmung der Mikro- und Makroereignisse im Zuge der Bewegungsanalyse der Bewegungsdaten ermöglicht.

Vorzugsweise umfassen die Gesundheitsdaten auch Informationen zu einer gesundheitsbedingten Bewegungsbeeinträchtigung des Hörsystemnutzers. Unter einer "gesundheitsbedingten Bewegungsbeeinträchtigung" ist hierbei insbesondere eine medizinische Erkrankung, ein gesundheitliches Handicap oder ein Gesundheitszustand des Hörsystemnutzers zu verstehen, welche(r) seine Bewegungen temporär beeinträchtigt oder einschränkt, und somit (zeitweise) eine Erhöhung eines Sturzrisikos bewirkt. Eine derartige Erkrankung kann beispielsweise eine Verletzung sein, welche die Verwendung einer Gehunterstützung oder Gehhilfe erfordert. Insbesondere ist hierbei auch eine Erkrankung in Form einer kognitiven Störung, insbesondere einer Störung des autonomen Nervensystems (ANS), wie beispielsweise ein Hörverlust, Demenz, Epilepsie oder Parkinson-Krankheit, zu verstehen.

Durch die hinterlegten Gesundheitsdaten ist es beispielsweise möglich, die Bewegungsmusteranalyse in Bezug auf Demenz oder Schwindel auszuführen, und somit eine genauere Bestimmung des Sturzrisikos zu ermöglichen. Insbesondere ist es somit möglich, das Warnsignal als Feedback rechtzeitig an den Hörsystemnutzer auszugeben.

Durch die hinterlegten Gesundheitsdaten wird für die Bewegungsanalyse und Risikobestimmung ein umfassenderes Bild des Hörsystemnutzers bereitgestellt, da sich nicht alle Informationen über eine ANS-Störung in ihrem spezifischen Gangbild befinden. Des Weiteren ist es möglich, dass ein Hörsystemnutzer an mehr als einer Störung leidet. Um also optimale Lösungen zu finden und falsche Zuschreibungen zu vermeiden, ist es notwendig möglichst viele verfügbaren Informationen auch jenseits von Gangmustern und deren Veränderungen bei der Risikobestimmung zu berücksichtigen. Des Weiteren wird somit die Stabilität und Zuverlässigkeit der Vorhersagen erhöht und Fehlklassifikationen im Falle von verrauschten Daten vermieden.

Falls verfügbar, können auch Daten von anderen, an das Hörsystem signaltechnisch gekoppelten, Sensoren wie Photoplethysmographie (PPG), Elektroenzephalographie (EEG), oder Elektromyographie (EMG) berücksichtigt werden, welche zum Beispiel Informationen über den kardiovaskulären Status (das heißt Frequenz, Herzfrequenzvariabilität) oder Belastungsmessungen liefern.

Erfindungsgemäß wird ein Hörgeräteparameter und/oder eine Hörgeräteleistung des Hörgeräts in Abhängigkeit der bestimmten Wahrscheinlichkeit eingestellt. Dies bedeutet, dass Einstellungen des Hörgeräts hinsichtlich der Signalverarbeitung und Ausgabe eines Umgebungssignals bei einer Änderung des Sturzrisikos variiert werden. Insbesondere werden die Einstellungen hierbei derart eingestellt oder geändert, dass das Sturzrisiko möglichst minimiert wird. Dadurch ist ein besonders sicherer und effektiver Sturzschutz realisiert.

Zusätzlich oder alternativ wird ein Hörgeräteparameter und/oder eine Hörgeräteleistung des Hörgeräts in Abhängigkeit der hinterlegten Gesundheitsdaten eingestellt. In einer geeigneten Weiterbildungsform wird beispielsweise die "Klassifizierung" für das automatische Hörgeräte-Programm je nach Benutzerprofil (Gesundheitsdaten) angepasst, was zu einer angepassten und geeigneten Hörgeräte-Ausgabe führt.

Beispielsweise leiden Menschen mit leichten kognitiven Beeinträchtigungen oder Demenz auch unter Konzentrationsschwäche, also unter einer Schwierigkeit den Fokus zu behalten oder unter einer leichten Ablenkbarkeit. Wenn durch die vom Hörgerät durchgeführte Ganganalyse ein Fortschreiten der leichten kognitiven Beeinträchtigung oder Demenz anhand eines erhöhten Sturzrisikos festgestellt wird, kann somit die Hörgeräteleistung zur besseren Orientierung in komplexen akustischen Umgebungen angepasst werden.

Beispielsweise wird ein Beamforming oder ein Beamform-Muster des Hörgeräts eingestellt. Unter "Beamforming" wird insbesondere eine Richtcharakteristik des Hörgeräts verstanden, dass also ein Richtungshören realisiert wird, so dass Schallquellen aus einer bestimmten Richtung relativ zu anderen Richtungen verstärkt werden. Hierzu wird ein Beamformer verwendet, welcher die Eingangssignale mehrerer unterschiedlicher Eingangswandler (Mikrofone) insbesondere desselben Einzelhörgeräts (Einzelgerät) in geeigneter Weise zu einem Ausgangssignal kombiniert.

Zum Ausgleich von Konzentrationsschwächen oder Ablenkbarkeit ist es beispielsweise möglich für die rückwärtige Hemisphäre die Kompression bei der Signalverarbeitung durch Absenken des ersten Kniepunktes anzupassen, und das Kompressionsverhältnis zu erhöhen. Das Kompressionssystem für die vordere Hemisphäre kann unverändert bleiben. Zusätzlich kann die Lärmreduktion für eine stärkere Wirkung parametrisiert werden. Diese beispielhafte Einstellung der Hörgeräteeinstellung/-parameter ermöglicht eine leichtere Fokussierung und weniger Ablenkungen durch akustischen Input aus rückwärtiger Richtung.

Im Bereich der Hörgeräte stellt die Kompression beispielsweise eine Möglichkeit dar, das Hörsignal an das eingeschränkte Hörvermögen eines Hörgeschädigten anzupassen. Das Hörvermögen eines Hörgeschädigten weist einen eingeschränkten Dynamikbereich auf, so dass kleine Eingangspegel viel verstärkt werden müssen. Für große Eingangspegel muss jedoch die Verstärkung abgesenkt werden, weil die derart verstärkten Hörsignale sonst als unangenehm laut empfunden werden. Die Kompression der Dynamik des Audio-Signals tritt dem hierbei durch eine Verstärkung mit einem pegelabhängigen Verstärkungsfaktor entgegen. In den meisten Fällen weist dabei die Kompressionscharakteristik, dargestellt durch die Kennlinie, welche das Verhältnis von Eingangspegel zu Ausgangspegel angibt, eine bis zu einem bestimmten Schwellwert des Eingangspegels lineare Verstärkung um einen konstanten Faktor auf, während für Eingangspegel jenseits des Schwellwerts die Verstärkung pegelabhängig gesenkt wird. Trägt man Eingangspegel und Ausgangspegel in Dezibel gegeneinander auf, so ergibt sich hierdurch als Kompressionscharakteristik ein streckenweise linearer Verlauf, wobei die Kennlinie ab dem Schwellwert für den Eingangspegel eine geringere Steigung aufweist (Kniepunkt).

Für eine optimale Behandlung von Hörverlust und ANS-Störungen werden somit die Hörsystemparameter, die die Hörgeräteleistung beeinflussen, entsprechend angepasst. Die Analyse kann auch zur Steuerung von Eingriffen verwendet werden, welche über ein an das Hörgerät signaltechnisch gekoppeltes Zusatzgerät, beispielsweise über ein angeschlossenes Smartphone, gesteuert werden, um ein rechtzeitiges Eingreifen zu ermöglichen, und um eine Umsetzung von individuellen Bedürfnisse des Hörsystemnutzers zu erleichtern sowie dem Fortschreiten altersbedingter Störungen entgegenzuwirken.

Vorzugsweise werden die Hörgeräteparameter, welche die Hörgeräteleistung beeinflussen, anhand des bestimmten Sturzrisikos angepasst. Der angepasste Hörgeräte-Ausgang (Ausgangssignal) könnte als Beitrag zu einer Intervention (zum Beispiel bei Demenz oder anderen Störungen) je nach Bedarf gesehen werden. Das resultierende veränderte Ausgangssignal ermöglicht beispielsweise eine bessere Orientierung oder eine Reduzierung von Distraktoren/Ablenkungen oder dergleichen. Hierzu wird im Rahmen der Signalverarbeitung beispielsweise eine Änderung von Multi-Source-Kompressions-Schemata (CK, CR, Verstärkung, Attack- & Release-Zeiten, Rauschunterdrückungs-Algorithmen) sowie von monauralen und/oder binauralen Beamformer-Muster vorgenommen.

In einer bevorzugten Ausgestaltung des Verfahrens wird anhand von Akustikdaten des Eingangswandlers (Eingangssignal) eine Umgebungssituation des Hörsystemnutzers bestimmt. Unter einer Umgebungssituation ist hierbei insbesondere eine akustische Umgebungssituation oder eine Hörsituation zu verstehen. Die Umgebungssituation wird hierbei beispielsweise mittels einer Situationserkennung und/oder mindestens einer Pegelmessung und/oder mindestens einen Algorithmus des Hörgeräts oder der Signalverarbeitung identifiziert und beschrieben. Beispielsweise wird die Umgebungssituation nach bestimmten Kriterien klassifiziert, und jeder dieser Klassen ist eine bestimmte Einstellung der Hörgeräteparameter und/oder Hörgeräteleistung zugeordnet.

Die aktuelle Umgebungssituation wird hierbei den jeweils aktuellen Bewegungsdaten zugeordnet und in einem Speicher hinterlegt. Dies bedeutet, dass für die Auswertung oder Risikobestimmung anhand der Bewegungsdaten auch die jeweilige Umgebung des Hörsystemnutzers berücksichtigt werden kann, so dass eine genauere und zuverlässigere Bestimmung des Sturzrisikos ermöglicht ist. Vorzugsweise erfolgt die Bestimmung der akustischen Umgebungssituation parallel zur Aufnahme der Bewegungsdaten.

In einer zweckmäßigen Ausführung werden für die Bestimmung der Wahrscheinlichkeit die Bewegungsdaten für eine jeweilige Umgebungssituation mit hinterlegten Bewegungsdaten und Umgebungssituationen verglichen. Mit anderen Worten erfolgt ein Vergleich mit vorhergehenden Analyseergebnissen und Bewegungsmustern in vergleichbaren und/oder verschiedenen Hörsituationen. Dadurch kann beispielsweise ein Sturzereignis oder Beinahe-Sturzereignis bei einer früheren Hörsituation für zukünftige Hörsituationen berücksichtigt werden, so dass in solchen Hörsituationen ein erhöhtes Sturzrisiko gegeben ist.

Zusätzlich oder alternativ ist beispielsweise ein Vergleich mit ähnlichen anderen Benutzerdaten, welche anonym in einer "Wolke" (Cloud), einem entfernten Server, oder einer Datenspeicherung gespeichert sind, möglich.

In einer vorteilhaften Ausbildung wird anhand der hinterlegten Bewegungsdaten und Umgebungssituationen ein Benutzerprofil für den Hörsystemnutzer erstellt. Mit anderen Worten wird der Hörsystemnutzers anhand der hinterlegten Daten zu einem Profil "klassifiziert". Dies ermöglicht eine tägliche Routineerkennung, um ein aktuelles Ergebnis mit früheren Ergebnissen zu vergleichen und somit Änderungen verfolgen und erfassen zu können. Durch das Benutzerprofil ist eine verbesserte und robustere Bestimmung des Sturzrisikos ermöglicht.

Ein zusätzlicher oder weiterer Aspekt der Erfindung sieht vor, dass das Hörgerät binaural ausgebildet ist und hierzu zwei Einzelgeräte aufweist, welche jeweils zumindest ein Eingangswandler sowie zumindest einen Ausgangswandler aufweisen und dadurch ausgebildet sind, Schallsignale aus der Umgebung aufzunehmen und an einen Nutzer des Hörgeräts auszugeben. Zusätzlich weist jedes der Einzelgeräte einen Bewegungssensor auf. Hierbei werden die Bewegungsdaten der Einzelgeräte getrennt voneinander ausgewertet, und anschließend zur Bestimmung der Wahrscheinlichkeit kombiniert werden.

Beispielsweise ist eine Drahtlosschnittstelle zum Datenaustausch zwischen den beiden Einzelgeräten vorgesehen. Die Drahtlosschnittstelle ist beispielsweise eine Bluetooth-Schnittstelle oder eine WLAN-Schnittstelle oder eine MI-Schnittstelle (MI-Link, MI: Magnetic Induction). Die Bluetooth-Schnittstelle ist beispielsweise eine reguläre oder eine Niedrigenergie (sogenannte low-energy) Bluetooth-Schnittstelle.

Alternativ zu einem binauralen Hörgerät ist aber auch ein monaurales Hörgerät mit lediglich einem Einzelgerät geeignet. Die Ausführungen bezüglich eines monauralen Hörgeräts sind sinngemäß auf ein binaurales Hörgerät übertragbar und umgekehrt.

Beispielsweise ist es ebenso denkbar, dass für die Daten der Einzelgeräte jeweils eine Wahrscheinlichkeit bestimmt wird. Vorzugsweise werden jedoch die Bewegungssensorinformationen beider Einzelgeräte getrennt analysiert und anschließend "kombiniert", wodurch auch komplexe Bewegungsmuster identifizierbar oder erfassbar sind, welche mit fortschreitenden ANS-Störungen verbunden sind. Dadurch wird die Bestimmung des Sturzrisikos weiter verbessert.

Bei einem binauralen Hörgerät werden die beiden Einzelgeräte vom Nutzer auf unterschiedlichen Seiten des Kopfs getragen, sodass jedes Einzelgerät einem Ohr zugeordnet ist. Somit sind der linke und der rechte Bewegungssensor (3D-Beschleunigungsmesser) in einem Abstand zur Körpermitte angeordnet, so dass die von beiden gelieferten Informationen kombiniert werden können, um tangentiale und radiale Kräfte zu charakterisieren, die eine Drehung anzeigen. Dadurch ist es beispielsweise möglich, einen Lagesensor oder ein Gyroskop einzusparen. Darüber hinaus ermöglichen die beiden unabhängigen Sensorausgänge eine erhöhte Zuverlässigkeit der Bewegungsdaten. Beispielsweise werden die Informationen für die Risikobewertung lediglich verwendet, wenn beide Bewegungssensoren das gleiche Ereignis oder die gleiche Änderung anzeigen.

In einer geeigneten Ausgestaltung werden die Bewegungsdaten und/oder die Wahrscheinlichkeit an ein signaltechnisch mit dem Hörgerät gekoppeltes Zusatzgerät übermittelt. Dadurch ist es beispielsweise möglich analysierte Symmetrien oder andere Schlüsselmetriken des Gehens, sowie Status und Änderungen in einer App, einer Website oder einer Software anzeigen zu lassen.

Bei dem Zusatzgerät handelt es sich vorzugsweise um ein mobiles Bedien- und Anzeigegerät, beispielsweise ein Mobiltelefon, insbesondere ein Mobiltelefon mit einer Computerfunktion beziehungsweise ein Smartphone oder auch ein Tabletcomputer. Das Zusatzgerät weist geeigneterweise eine hinterlegte Anwendungssoftware (Betriebssoftware) auf, mit welcher beispielsweise das Warnsignal erzeugt wird, wenn die Wahrscheinlichkeit den Schwellwert erreicht oder überschreitet. Die Anwendungssoftware (Application software) ist hierzu vorzugsweise als eine sogenannte App oder Mobile App (Mobilanwendung, Smartphone-App) auf dem Bedien- und Anzeigegerät installierbar beziehungsweise installiert.

Diese Weiterbildung geht dabei von der Überlegung aus, dass moderne Bedien- und Anzeigegeräte, wie insbesondere Smartphones oder Tabletcomputer, in der heutigen Gesellschaft weit verbreitet sind und einem Benutzer generell jederzeit verfügbar und zugänglich ist. Insbesondere weist der Benutzer des Hörsystems mit großer Wahrscheinlichkeit im Wesentlichen ein derartiges Bedien- und Anzeigegerät in seinem Haushalt auf.

Moderne Smartphones sind heutzutage weiterhin standardmäßig mit einer Vielzahl an unterschiedlichen Nahfeld- und Fernfeldkommunikationsmitteln ausgerüstet, wodurch eine Kommunikations- oder Signalverbindung zu dem Hörgerät prinzipiell auf einfache Weise herstellbar ist. Die Anwendungssoftware ist hierbei vorzugsweise auch zur Einstellung von Betriebsparametern des Hörgeräts, wie beispielsweise einer Lautstärke, geeignet und eingerichtet. Dadurch benötigt der Benutzer kein zusätzliches, separates Bediensystem zur Überwachung und Einstellung des Hörsystems, sondern es ist möglich, durch ein (nachträgliches) Herunterladen und/oder Installieren der Anwendungssoftware sein bereits vorhandenes Smartphone zur Auswertung der Bewegungsdaten und zur Bestimmung des Sturzrisikos zu verwenden. Auf diese Weise werden benutzerseitige Kosten vorteilhaft reduziert. Hierbei ist es beispielsweise denkbar, dass die Gesundheitsdaten in einem Speicher des Zusatzgeräts beziehungsweise Smartphones hinterlegt sind.

Die typischerweise als Touchscreens (Anzeige, Display) ausgebildeten Oberflächen von Smartphones oder Tabletcomputern erlauben weiterhin eine besonders einfache und intuitive Bedienung der Anwendungssoftware des dadurch gebildeten Zusatzgeräts. Dadurch ist ein Smartphone oder Tabletcomputer besonders kostengünstig für die Überwachung des Sturzrisikos nachrüstbar.

Das Bedien- und Anzeigegerät umfasst einen internen Controller, welcher zumindest im Kern durch einen Mikrocontroller mit einem Prozessor und einem Datenspeicher gebildet ist, in dem die Funktionalität zur Durchführung des Verfahrens in Form der Anwendungssoftware programmtechnisch implementiert ist, so dass das Verfahren beziehungsweise die Bestimmung des Sturzrisikos- gegebenenfalls in Interaktion mit dem Benutzer - bei Ausführung der Anwendungssoftware in dem Mikrocontroller automatisch durchgeführt wird.

Das Zusatzgerät kann dazu ausgebildet sein, Daten eines digitalen Assistenten, welcher von der externen Datenquelle bereitgestellt wird, an das Hörgerät zu übertragen. Unter einem digitalen Assistenten wird insbesondere eine Sprachsteuerung verstanden, welche Sprachbefehle des Nutzers erkennt und je nach Sprachbefehl verschiedene Funktionen oder Aktionen ausführen kann und auch ausführt. Mit anderen Worten: der digitale Assistent ist insbesondere ausgebildet zur Spracherkennung, zur Sprachanalyse, zur Suche von Informationen oder zum Abarbeiten einfacher Aufgaben oder einer Kombination hiervon. Der digitale Assistent bildet insbesondere eine Schnittstelle zwischen dem Nutzer und einem anderen Gerät, insbesondere der externen Datenquelle. Insbesondere gibt der digitale Assistent Daten aus, welche für den Nutzer bestimmt sind, insbesondere in Form von Sprache. Diese Daten, d.h. eine Ausgabe des digitalen Assistenten und speziell eine Sprachausgabe des digitalen Assistenten, wird dann über das Zusatzgerät an das Hörgerät übertragen, zur Ausgabe an den Nutzer. Auf diese Weise werden vorteilhaft ein Feedback und eine Informationsausgabe vom digitalen Assistenten an den Nutzer ermöglicht. Die externe Datenquelle ist speziell hierbei, aber auch allgemein vorzugsweise die Cloud.

Über das Zusatzgerät ist es somit beispielsweise möglich, Benutzer-Feedback basierend auf dem Analyse- oder Risikoergebnis anzufordern. Das Benutzer-Feedback könnte hierbei einerseits die vom Hörsystem bei unsicherem Analyseergebnis ausgeführte Aktion verfeinern und/oder andererseits eine algorithmische Verbesserung der Bewegungsanalyse ermöglichen, indem ein Label für die aufgezeichnete Bewegung erfasst wird. Hierbei "lernt" das Hörsystem, spezifische Bewegungsvariationen für den individuellen Benutzer zu erkennen oder die Klassifizierung von Bewegungen für die allgemeine Bevölkerung auf der Grundlage des Benutzerfeedbacks zu verbessern. Die Rückkopplung kann durch eine App, durch Sprachbefehle oder andere Interaktionen mit dem Hörsystem erfasst werden.

Die Analyse-Ergebnisse können auch dem Gesundheitsversorger und/oder einem medizinischen Experten zur Verfügung gestellt werden, um die einmalige Diagnostik und die kontinuierliche Überwachung von altersbedingten Störungen zu unterstützen, sowie um spezifische Veränderungen im Bewegungsmuster frühzeitig zu erkennen.

Die Analyse der Bewegungsdaten kann in dem Hörgerät und/oder ferngesteuert in dem an das Hörgerät gekoppelten Zusatzgerät beziehungsweise Smartphone durchgeführt werden. Im letzteren Fall werden vorzugsweise die rohen oder vorverarbeiteten Bewegungsdaten an das angeschlossene Smartphone gesendet. Wenn die Analyse auf dem Smartphone durchgeführt wird, ist es notwendig, das Ergebnis für das Sturzrisiko an das Hörgerät zurückzusenden. Beispielsweise ist es ebenso möglich, dass auch der Schwellwertvergleich von dem Zusatzgerät ausgeführt wird, wobei das Zusatzgerät das Warnsignal erzeugt und/oder ein das Warnsignal auslösendes Signal an das Hörgerät übermittelt.

Mit anderen Worten besteht eine Möglichkeit zur Durchführung des Verfahrens darin, dass die notwendige Verarbeitung und Auswertung der Bewegungsdaten zur Risikobestimmung in dem Hörgerät erfolgt. Eine andere Möglichkeit besteht darin, dass das Hörgerät lediglich die relevanten Bewegungs-/Akustikdaten sammelt, diese optional in einem Puffer speichert, und die Rohdaten (oder vorverarbeitete Daten) zur abschließenden Analyse an das (hörgerät-)externe Zusatzgerät beziehungsweise Smartphone des Hörsystems überträgt. Dabei könnte sowohl die Rechenleistung des Smartphones als auch von Cloud-Diensten genutzt werden.

Daraus ergibt sich insbesondere der Vorteil, dass das Hörgerät entlastet wird, so dass die Anforderungen an die Signalverarbeitung oder Elektronik im Hörgerät vorteilhaft reduziert werden. Die Funktionalität der Bewegungsanalyse, insbesondere der Risikobestimmung, ist somit ganz oder teilweise an das Zusatzgerät übertragbar, sodass das Hörgerät entsprechend Energie spart.

In einer denkbaren Ausführung des Verfahrens werden beim Erreichen oder Überschreiten des Schwellwerts auf dem Zusatzgerät Maßnahmen zur Reduzierung eines Sturz- oder Fallrisikos angezeigt. Mit anderen Worten werden dem Hörsystemnutzer Hinweise oder Vorschläge auf dem Zusatzgerät angezeigt, welche das Sturzrisiko vermindern sollen. Diese Maßnahmen werden insbesondere auch als Beratung oder Counseling bezeichnet. Ein Grundgedanke ist hierbei, dem Anwender Hinweise und Vorschläge zu unterbreiten, welche zu einer weiteren Reduzierung des Sturzrisikos beitragen, welche aber nicht in der Einflusssphäre des Hörgeräts liegen.

Beispielsweise werden die Bewegungsdaten (inkl. Analyse) an das Zusatzgerät oder Smartphone gesendet, um Behandlungen und Tipps anzubieten, welche von der Smartphone-App bereitgestellt werden. Basierend auf der Gang-/Risikoanalyse empfiehlt das Smartphone beispielsweise Aktionen, die helfen sollen eine Erhaltung oder Erhöhung der körperlichen Fitness zur Verringerung des Sturzrisikos, oder eine Verlangsamung eines kognitiven Verfalls, oder ein gesundes Altern oder gute Lebensqualität zu ermöglichen, oder eine Unterstützung anderer Interventionen, zum Beispiel Dosen von pharmazeutischen Interventionen, zu unterstützen.

Weiterhin ist es beispielsweise denkbar, dass der Hörsystemnutzers über seine Smartphone-App tägliche Updates und Tipps zum gesunden Altern, Kochrezepte et cetera erhält. Wenn die Ausgabe einer vom Hörgerät durchgeführten Ganganalyse auf eine Verschlechterung einer bekannten Störung, eine Veränderung des Bewegungsmusters in Verbindung mit erhöhter Gebrechlichkeit oder eine Erhöhung des Sturzrisikos hinweist, könnte der Inhalt der Tipps der Smartphone-App angepasst werden, um das aktuelle Niveau der Lebensqualität zu erhalten oder zu erhöhen, um somit ein möglichst lebenslanges autonomes Leben zu ermöglichen. Zum Beispiel könnte die Smartphone-App hierbei Maßnahmen empfehlen, die helfen, die weitere Entwicklung der Gebrechlichkeit zu verhindern (d.h. Übungen, Spaziergänge etc.), eine Ernährungsumstellung zur Vermeidung von Nebenwirkungen, oder über die Notwendigkeit eines Arztbesuches zur Anpassung der Medikation et cetera informieren. Auf diese Weise könnte das Hörsystem dazu beitragen, hohe Kosten im Gesundheitssystem zu vermeiden, indem es pro-aktiv dazu beiträgt, die Menschen gesünder, mobiler und selbständiger zu halten.

In einer denkbaren Ausbildung des Verfahrens werden die Bewegungsdaten zur Bestimmung der (Sturz-)Wahrscheinlichkeit während eines vorgegebenen Bewegungsmusters des Hörsystemnutzers erfasst. Insbesondere wird während der Erfassung ein vorgegebenes Bewegungsprotokoll von dem Hörsystemnutzer ausgeführt. Diese Ausbildung ist insbesondere im Bereich von klinischen, diagnostischen oder therapeutischen Anwendungen nützlich. Insbesondere ist somit ein besserer Abgleich zu anderen Patienten möglich, so dass eine Verbesserung der Sturzrisikobestimmung ermöglicht ist.

Vorteilhafterweise wird die Bewegungsanalyse hierbei explizit durch den Benutzer ausgelöst, des Weiteren findet die Bewegungsanalyse insbesondere in einer kontrollierten Umgebung mit einem klaren Erwartungshorizont über die bevorstehende Bewegung statt. Dadurch kann eine Abtastung und Verarbeitung von Bewegungsmustern intensiviert werden, wobei anschließend also nach den Übungen ein normaler Verarbeitungsmodus verwendet wird, um den Stromverbrauch zu reduzieren. Mit anderen Worten findet während des vorgegebenen Bewegungsmusters vorzugsweise eine Erfassung der Bewegungsdaten mit einer höheren Auflösung oder höheren Messrate statt, so dass auch geringe Abweichungen oder Störungen zuverlässig erkannt werden. Dadurch ist eine Früherkennung für Verschlechterungen von ANS-Störungen verbessert, so dass eine besonders zuverlässige Sturzsicherung ermöglicht ist.

Für die Analyse von Bewegungsmustern werden hierbei beispielsweise mehrere Bewegungs-Kategorien abgedeckt. Beispielsweise werden "stationäre Übungen", welche vom Patienten/Benutzer entweder allein oder unter Aufsicht eines Krankenpflegers oder Therapeuten durchgeführt werden können, idealerweise in einer täglichen Routine ausgeführt. Diese Übungen können App-geführt sein, dies bedeutet, dass die Übungen beispielsweise auf dem Zusatzgerät angezeigt und vom Hörsystemnutzer ausgeführt werden. Mögliche Übungen (aber keine vollständige Liste) sind: Stuhl-Aufsteh-Test, Schwingungsanalyse, Aufheben von Gegenständen, Schuhbinden, Hinlegen / Aufstehen, Umdrehen.

Zusätzlich oder alternativ erfolgt außerhalb der vorgegebenen Bewegungsmuster eine permanente, kontinuierliche Überwachung, bei welcher alle Bewegungsmuster ständig analysiert, bewertet und kategorisiert werden. Dies ist vorteilhaft, um ein Benutzerprofil und die Gesamtverteilung der bewegungsbezogenen Aktivitäten zu erstellen. Es benötigt weniger Benutzerinteraktion, ist jedoch vergleichsweise rechenintensiv. Daher ist auch eine kontinuierliche Überwachung für ein bestimmtes Zeitintervall denkbar, welche beispielsweise durch ein Schlüsselereignis, zeitbasiert oder auf Benutzeranforderung ausgelöst werden kann.

Vorzugsweise werden Umgebungs-/Bewegungsmuster kontinuierlich für Grundbewegungen, zum Beispiel Gehen, analysiert. Wenn das Gehen erkannt wird, wird die nächste Verarbeitungshierarchie aktiviert, die den Gang und seine Symmetrie genauer analysiert. Dies kann für eine bestimmte Zeitspanne, zum Beispiel einige Minuten, oder bis zum Ende des Gehens erfolgen. Es könnte so konzipiert werden, dass diese Bewegungsanalyse für eine definierte oder vorgebbare Anzahl am Tag durchgeführt wird.

Das erfindungsgemäße Hörsystem ist insbesondere als eine Hörhilfevorrichtung ausgeführt, und weist ein Hörgerät auf. Das Hörgerät weist mindestens einen Eingangswandler zum Empfangen eines akustischen Umgebungssignals, und einen Ausgangswandler zur Ausgabe eines akustischen Signals, sowie einen Bewegungssensor zum Erfassen einer Körperbewegung eines Hörsystemnutzers auf.

Das Hörsystem weist weiterhin einen Controller, also eine Steuereinheit, auf. Der Controller ist beispielsweise in das Hörgerät integriert und ist zum Beispiel Teil einer Signalverarbeitung. Zusätzlich oder alternativ ist es ebenso denkbar, dass der Controller ein Teil eines signaltechnisch mit dem Hörgerät gekoppelten oder koppelbaren Zusatzgeräts, insbesondere eines Smartphones, ist.

Der Controller ist hierbei allgemein - programm- und/oder schaltungstechnisch - zur Durchführung des vorstehend beschriebenen erfindungsgemäßen Verfahrens eingerichtet. Der Controller ist somit konkret dazu eingerichtet, eine Benutzerbewegung oder ein Bewegungsereignis zu analysieren oder zu charakterisieren, um somit insbesondere ein Fortschreiten altersbedingter Störungen und die damit verbundene Veränderung des Sturzrisikos zu analysieren.

In einer bevorzugten Ausgestaltungsform ist der Controller zumindest im Kern durch einen Mikrocontroller mit einem Prozessor und einem Datenspeicher gebildet, in dem die Funktionalität zur Durchführung des erfindungsgemäßen Verfahrens in Form einer Betriebssoftware (Firmware) programmtechnisch implementiert ist, so dass das Verfahren - gegebenenfalls in Interaktion mit einem Vorrichtungsnutzer - bei Ausführung der Betriebssoftware in dem Mikrocontroller automatisch durchgeführt wird. Der Controller kann im Rahmen der Erfindung alternativ aber auch durch ein nicht-programmierbares elektronisches Bauteil, wie zum Beispiel einem anwendungsspezifischen integrierten Schaltkreis (ASIC), gebildet sein, in dem die Funktionalität zur Durchführung des erfindungsgemäßen Verfahrens mit schaltungstechnischen Mitteln implementiert ist.

Nachfolgend sind Ausführungsbeispiele der Erfindung anhand einer Zeichnung näher erläutert. Darin zeigen in schematischen und vereinfachten Darstellungen:
- Fig. 1: ein Hörsystem mit einem binauralen Hörgerät,
- Fig. 2: das Hörsystem gemäß Fig. 1, bei welchem das Hörgerät signaltechnisch mit einem mobilen Zusatzgerät gekoppelt ist, und
- Fig. 3: ein Flussdiagramm eines Verfahrens zum Betreiben des Hörsystems,
- Fig. 4: zwei Zeit-Beschleunigungs-Diagramme für einen Sitz-Aufsteh-Test,
- Fig. 5: zwei Zeit-Beschleunigungs-Diagramme für einen Umdreh-Test, und
- Fig. 6: drei Beschleunigungs-Beschleunigungs-Diagramme für einen Stillsteh-Test.

Einander entsprechende Teile und Größen sind in allen Figuren stets mit den gleichen Bezugszeichen versehen.

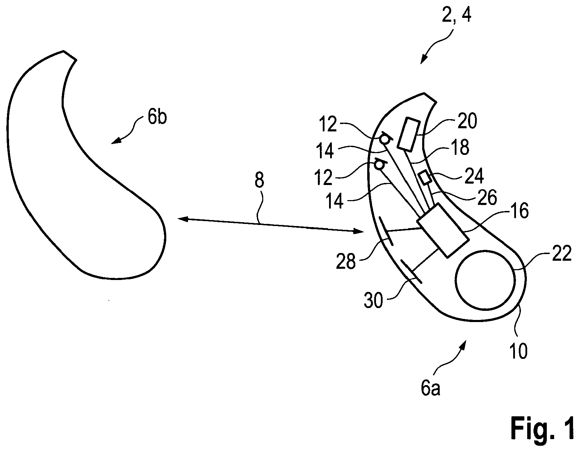

Die Fig. 1 zeigt den prinzipiellen Aufbau eines erfindungsgemäßen Hörsystems 2. In diesem Ausführungsbeispiel ist das Hörsystem 2 als eine Hörhilfevorrichtung mit einem binauralen Hörgerät 4 mit zwei signaltechnisch gekoppelten Hörhilfegeräten beziehungsweise Einzelgeräten 6a, 6b ausgeführt. Die Einzelgeräte 6a, 6b sind hierbei beispielhaft als Hinter-dem-Ohr-Hörhilfegeräte (HdO) ausgestaltet. Die Einzelgeräte 6a, 6b sind untereinander mittels einer drahtlosen Kommunikationsverbindung 8 signaltechnisch gekoppelt oder koppelbar.

Die Kommunikationsverbindung 8 ist beispielsweise eine induktive Kopplung zwischen den Einzelgeräten 6a und 6b, alternativ ist die Kommunikationsverbindung 8 beispielsweise als eine Funkverbindung, insbesondere als eine Bluetooth- oder RFID-Verbindung, zwischen den Einzelgeräten 6a und 6b ausgeführt ist.

Der Aufbau der Einzelgeräte 6a, 6b ist nachfolgend beispielhaft anhand des Einzelgeräts 6a erläutert. Das Einzelgeräte 6a umfasst, wie in der Fig. 1 schematisch dargestellt, ein Gerätegehäuse 10, in welches ein oder mehrere Mikrofone, auch als akusto-elektrische Eingangswandler 12 bezeichnet, eingebaut sind. Mit den Eingangswandlern 12 wird ein Schall beziehungsweise die akustischen Signale in einer Umgebung des Hörsystems 2 aufgenommen und in ein elektrisches Audiosignal als Akustikdaten 14 gewandelt.

Die Akustikdaten 14 werden von einer Signalverarbeitungseinrichtung 16, welche ebenfalls in dem Gerätegehäuse 10 angeordnet ist, verarbeitet. Anhand des Audiosignals 14 erzeugt die Signalverarbeitungseinrichtung 16 ein Ausgangssignal 18, welches an einen Lautsprecher beziehungsweise Hörer 20 geleitet wird. Der Hörer 20 ist hierbei als ein elektro-akustischer Ausgangswandler 20 ausgeführt, welcher das elektrische Ausgangssignal 18 in ein akustisches Signal wandelt und ausgibt. Bei dem HdO-Einzelgerät 6a wird das akustische Signal gegebenenfalls über einen nicht näher dargestellten Schallschlauch oder externen Hörer, der mit einer im Gehörgang einsitzenden Otoplastik, zum Trommelfell eines Hörsystemnutzers übertragen. Es ist aber auch beispielsweise ein elektro-mechanischer Ausgangswandler als Hörer 20 denkbar, wie beispielsweise bei einem Knochenleitungshörer.

Die Energieversorgung des Einzelgeräts 6a und insbesondere der Signalverarbeitungseinrichtung 16 erfolgt mittels einer in dem Gerätegehäuse 10 aufgenommenen Batterie 22.

Die Signalverarbeitungseinrichtung 16 ist mit einem Bewegungssensor 24 des Einzelgeräts 6a gekoppelt. Der Bewegungssensor 24 erfasst im Betrieb Beschleunigungs- und/oder Rotationsbewegungen des Einzelgeräts 6a und sendet diese im Betrieb als Bewegungsdaten 26 an die Signalverarbeitungseinrichtung 16. Der Bewegungssensor 24 ist beispielsweise als ein 3D-Beschleunigungssensor ausgeführt. Zusätzlich oder alternativ ist der Bewegungssensor beispielsweise als ein Lage-Sensor, insbesondere als ein gyroskopischer Sensor, ausgeführt.

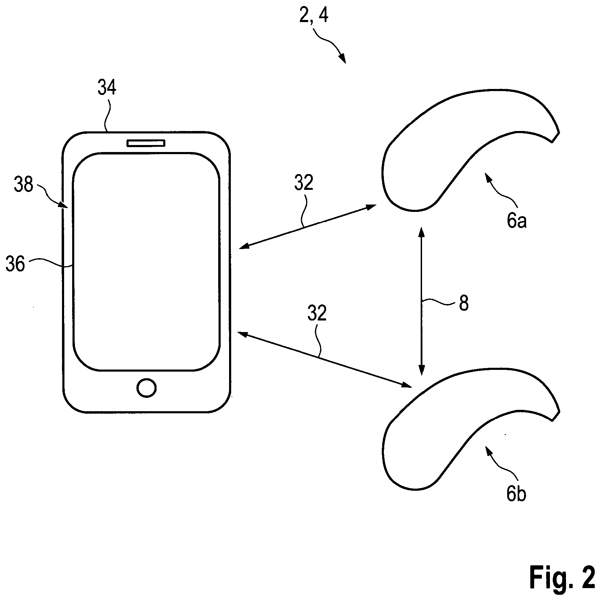

Die Signalverarbeitungseinrichtung 16 ist weiterhin signaltechnisch an einen ersten Transceiver 28 und an einen zweiten Transceiver 30 des Einzelgeräts 6a geführt. Der Transceiver 28 dient zum Senden und Empfangen von drahtlosen Signalen mittels der Kommunikationsverbindung 8 und der Transceiver 30 zum Senden und Empfangen von drahtlosen Signalen mittels einer Kommunikationsverbindung 32 an ein hörgeräteexternes Zusatzgerät 34 (Fig. 2). Beispielsweise ist es ebenso denkbar, dass lediglich ein Transceiver für beide Kommunikationsverbindungen 8, 32 vorgesehen ist.

In dem Ausführungsbeispiel der Fig. 2 ist das Zusatzgerät 34 als ein separates, mobiles, Bedien- und Anzeigegerät ausgeführt, welches mittels der Kommunikationsverbindung 32 signaltechnisch mit Hörgerät 4 gekoppelt oder koppelbar ist. Bei dem in Fig. 2 schematisch dargestellten Zusatzgerät 34 handelt es sich insbesondere um ein Smartphone. Das nachfolgend auch als Smartphone bezeichnete Zusatzgerät 34 weist eine berührungssensitive Anzeigeeinheit (Display) 36 auf, welche nachfolgend auch als Touchscreen bezeichnet wird. Zweckmäßigerweise ist das Smartphone 34 hierbei in den Sendebereich der Kommunikationsverbindung 32 eingebracht. Die signaltechnische Kopplung zwischen dem Smartphone 32 und den Transceivern 30 der Einzelgeräte 6a und 6b erfolgt hierbei über einen entsprechenden - nicht näher bezeichneten - integrierten Transceiver, beispielsweise einer Funk- oder Radioantenne, des Smartphones 32.

Das Smartphone 34 weist einen integrierten Controller auf, welcher im Wesentlichen durch einen Mikrocontroller mit einer implementierten Anwendungssoftware 38 zur programmtechnischen Auswertung von mittels der Kommunikationsverbindung 32 übermittelten Signalen und Daten gebildet ist. Die Anwendungssoftware 38 ist vorzugsweise eine Mobile-App beziehungsweise eine Smartphone-App, die in einem Datenspeicher des Controllers hinterlegt ist. Der Controller stellt im Betrieb die Anwendungssoftware 38 auf der als Touchscreen ausgeführten Anzeigeeinheit 36 dar, wobei die Anwendungssoftware 38 mittels der berührungssensitiven Oberfläche der Anzeigeeinheit 36 durch einen Hörsystemnutzers 2 bedienbar ist.

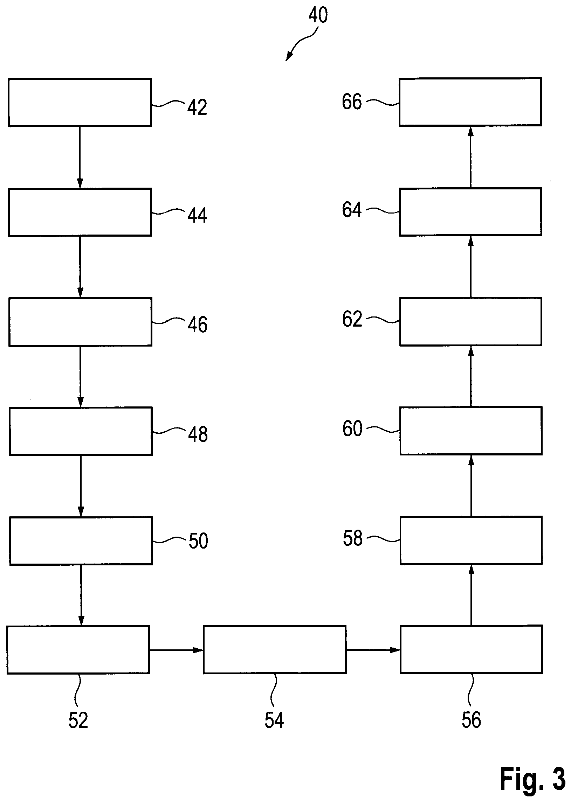

Anhand des in Fig. 3 dargestellten Flussdiagramms ist nachfolgend ein erfindungsgemäßes Verfahren 40 zum Betreiben des Hörsystems 2 näher erläutert.

Das Verfahren 40 ist insbesondere dazu geeignet und eingerichtet einen Hörsystemnutzer 2 vor einem sich anbahnenden Sturz zu schützen oder zu warnen. Im Normalbetrieb der Hörhilfevorrichtung 2 werden die Einzelgeräte 6a und 6b des Hörgeräts 4 an den Ohren des Hörsystemnutzers getragen. Hierbei sind die Einzelgeräte 6a und 6b mittels der Kommunikationsverbindungen 8 für eine gegenseitige Signalübertragung gekoppelt. Die Einzelgeräte 6a und 6b sind hierbei weiterhin optional mittels der Kommunikationsverbindung 32 signaltechnisch mit dem Smartphone 34 gekoppelt.

Verfahrensgemäß wird zunächst eine Bewegung des Hörsystemnutzers 2 oder ein Bewegungsereignis analysiert und/oder charakterisiert. Anhand der Auswertung oder Analyse wird eine Wahrscheinlichkeit für ein zukünftiges Sturzereignis, also ein Sturzrisiko, für den Hörsystemnutzers bestimmt. Dieses Sturzrisiko wird mit einem hinterlegten Schwellwert verglichen, und ein wahrnehmbares Warnsignal erzeugt, wenn der Schwellwert erreicht oder überschritten wird. Das Warnsignal ist beispielsweise ein vom Hörgerät 4 und/oder dem Smartphone 34 erzeugter Warnton. Zusätzlich oder alternativ ist das Warnsignal beispielsweise als eine Push-Benachrichtigung und/oder ein haptisches Vibrationssignal des Smartphones 34 ausgeführt.

Das Verfahren wird vorzugsweise zumindest teilweise von einem Controller ausgeführt, wobei das Verfahren beispielsweise im Hörgerät 4 und/oder in dem Smartphone 34 ausgeführt wird. Mit anderen Worten ist der Controller beispielsweise Teil der Signalverarbeitung 16 oder des Smartphones 34. Ebenso denkbar ist beispielsweise, dass das Verfahren teilweise im Hörgerät 4 und teilweise im Smartphone 34 ausgeführt wird, also dass sowohl das Hörgerät 4 als auch das Smartphone 34 einen solchen Controller aufweisen. Hierzu werden Daten über die Kommunikationsverbindung 32 vom Hörgerät 4 zu dem Smartphone 34 und zurück versendet.

Die Analyse und Auswertung der Bewegung kann beispielsweise in mehreren Schritten erfolgen, so dass eine besonders sichere und zuverlässige Bestimmung des Sturzrisikos ermöglicht ist. Die Fig. 3 zeigt hierbei eine aufeinanderfolgende Reihenfolge mehrerer Verfahrensschritte, wobei die Schritte hierbei beispielsweise zumindest teilweise in einer anderen Reihenfolge und/oder auch zumindest teilweise parallel ausgeführt werden können.

Beispielsweise werden in einem Verfahrensschritt 42 zunächst die Bewegungsdaten 26 erfasst und ausgewertet. Dies bedeutet, dass die Bewegung des Hörsystemnutzers 2 in einer aktuellen Situation unter Verwendung des Bewegungssensors 24 analysiert wird. Die Analyse oder Auswertung erfolgt beispielsweise für beide Einzelgeräte 6a, 6b getrennt oder es werden die Bewegungsdaten 26 von beiden Einzelgeräten 6a, 6b kombiniert analysiert.

In einem Verfahrensschritt 44 wird anhand der Audiosignale 14 die aktuelle akustische Umgebungssituation analysiert. Diese Analyse oder Auswertung erfolgt vorzugsweise parallel zur Auswertung der Bewegungsdaten 26. Geeigneterweise wird die jeweils aktuelle Umgebungssituation den aktuellen Bewegungsdaten 26 zugeordnet und in einem nicht näher dargestellten Speicher hinterlegt.

In einem Verfahrensschritt 46 werden die aktuellen Bewegungsdaten 26 und Umgebungssituation beispielsweise mit vorhergehenden beziehungsweise hinterlegten Bewegungsdaten 26 in vergleichbaren und verschiedenen Umgebungssituationen verglichen, um beispielsweise Abweichungen oder Abnormalitäten der Bewegungsmuster festzustellen. Derartige Abweichungen oder Abnormalitäten sind ein Hinweis auf ein erhöhtes Sturzrisiko.

In einem optionalen Verfahrensschritt 48 wird beispielsweise ein Benutzer-Feedback basierend auf dem Ergebnis des Verfahrensschritts 44 angefordert. Dies erfolgt beispielsweise mittels der Anwendungssoftware und/oder mittels Sprachbefehlen. Dieses Feedback wird beispielsweise angefordert, wenn ein unbekanntes Bewegungsmuster und/oder eine unbekannte akustische Umgebungssituation erkannt wird.

Vorzugsweise sind in einem Speicher des Hörsystems 2 persönliche Gesundheitsdaten des Hörsystemnutzers hinterlegt. In einem Verfahrensschritt 50 werden diese Gesundheitsdaten bei der Bewegungsmusteranalyse berücksichtigt. Beispielsweise erfolgt hierbei eine Bewegungsmusteranalyse der Bewegungsdaten 26 in Bezug auf Demenz oder Schwindel.

In dem optionalen Verfahrensschritt 52 wird - falls verfügbar - ein Input oder eine Eingabe von weiteren an das Hörsystem 2 gekoppelten Sensoren, wie beispielsweise Photoplethysmographie (PPG), Elektroenzephalographie (EEG), Elektromyographie (EMG), erfasst, welche zum Beispiel Informationen über den kardiovaskulären Status (d.h. Frequenz, Herzfrequenzvariabilität) des Hörsystemnutzers 2 und/oder Belastungsmessungen liefern.

In einem ebenfalls optionalen Verfahrensschritt 54 werden die aktuellen Bewegungsdaten 26 mit ähnlichen anderen Benutzerdaten verglichen, welche anonym, also einem Benutzer nicht zuordenbar, in einer Cloud gespeichert sind. Der Verfahrensschritt 54 entspricht hierbei beispielsweise dem Verfahrensschritt 46, wobei es sich hierbei nicht um hinterlegte Daten des Hörsystemnutzers 2 selbst handelt, sondern um Daten anderer Benutzer welche in der Cloud abrufbar sind. Dadurch können vergleichbare Daten anderer Benutzer zur Bestimmung des Sturzrisikos mitberücksichtigt werden, wodurch eine robustere und verlässlichere Voraussage der Sturz-Wahrscheinlichkeit gegeben ist.

In einem Verfahrensschritt 56 wird anhand der hinterlegten Bewegungsdaten und Umgebungssituationen ein Benutzerprofil für den Hörsystemnutzer erstellt. Mit anderen Worten wird der Hörsystemnutzers anhand der hinterlegten Daten zu einem Profil "klassifiziert". Dies ermöglicht eine tägliche Routineerkennung, um ein aktuelles Ergebnis mit früheren Ergebnissen zu vergleichen und somit Änderungen verfolgen und erfassen zu können. Durch das Benutzerprofil ist eine verbesserte und robustere Bestimmung des Sturzrisikos ermöglicht.

In einem Verfahrensschritt 58 werden die Hörgeräteeinstellungen, also Hörgeräteparameter und/oder Hörgerätleistung, in Abhängigkeit der Verfahrensschritte 46 und/oder 54 angepasst. Der angepasste Hörgeräte-Ausgang kann als Beitrag zu einer Sturz-Intervention oder Sturz-Prävention (z.B. bei Demenz oder anderen Störungen) genutzt werden. Die Veränderung der Hörgeräteeinstellungen kann zu einer verbesserten Orientierung und/oder zur Reduzierung von Distraktoren beitragen, und somit das Sturzrisiko senken. Beispielsweise wird im Verfahrensschritt 58 das aktuell bestimmte Sturzrisiko mit einem (zweiten) Schwellwert verglichen, welcher kleiner dimensioniert ist als der (erste) Schwellwert für die Auslösung des Warnsignals. Wird dieser (zweite) Schwellwert erreicht oder überschritten werden die Hörgeräteeinstellungen verändert und angepasst. Dadurch wird vor der Ausgabe des Warnsignals zunächst eine weitere präventive Maßnahme zu Reduzierung des Sturzrisikos ausgeführt. Vorzugsweise wird somit eine korrelative oder graduelle Anpassung der Hörgeräteeinstellungen mit zunehmender Sturzwahrscheinlichkeit realisiert.

In einer denkbaren Ausführung des Verfahrens werden beim Erreichen oder Überschreiten des (ersten oder zweiten) Schwellwerts auf dem Zusatzgerät Maßnahmen zur Reduzierung des Sturz- oder Fallrisikos angezeigt. In einem Verfahrensschritt 60 werden dem Hörsystemnutzer 2 hierzu in Abhängigkeit der Bewegungsdaten 26 und/oder des Sturzrisikos Behandlungen oder Tipps angeboten, welche von der Anwendungssoftware 38 des Smartphones 34 bereitgestellt werden. Beispielsweise werden basierend auf der Ganganalyse auf der Anzeigeeinheit 36 Aktionen angezeigt, welche zu einer Erhaltung oder Erhöhung der körperlichen Fitness zur Verringerung des Sturzrisikos beitragen, oder einen kognitiven Verfall verlangsamen, oder ein gesundes Altern oder eine gute Lebensqualität ermöglichen, oder andere Interventionen unterstützen, beispielsweise eine Einnahme von Dosen von pharmazeutischen Interventionen.

In einem Verfahrensschritt 62 werden anhand der ausgewerteten Bewegungsdaten 26 Änderungen in den Bewegungsmustern und/oder des Sturzrisikos sowie ein aktueller Status auf der Anzeigeeinheit 36 des Smartphones 34 gezeigt. Dadurch kann der Hörsystemnutzer 2 sein Sturzrisiko selbst überwachen. Dies ist insbesondere vorteilhaft, wenn über das Verfahren eine weitere ANS-Störung des Hörsystemnutzers 2 überwacht wird, so dass der Hörsystemnutzers 2 auch ohne einen Arztbesuch seinen Gesundheitszustand oder Krankheitsverlauf überwachen kann.

In einem Verfahrensschritt 64 wird eine "Klassifizierung" für das Hörgerät je nach Benutzerprofil angepasst, wodurch eine verbesserte Ausgabe für den Hörsystemnutzers 2 bereitgestellt wird. Dies bedeutet, dass anhand des im Verfahrensschritt 56 erstellten Benutzerprofils die hinterlegten Voreinstellungen des Hörgeräts 4 für unterschiedliche Umgebungssituationen angepasst werden, um das Sturzrisiko in diesen Situationen möglichst gering zu halten. Im Gegensatz zu dem Verfahrensschritt 58, bei welchem die aktuellen Hörgeräteeinstellungen angepasst werden, werden im Verfahrensschritt 64 die Hörgeräteeinstellungen, welche den unterschiedlichen akustischen Umgebungssituationen zugeordnet sind, modifiziert. Dadurch werden bei einem zukünftigen Umgebungswechsel bereits verbesserte Hörgeräteeinstellungen hinsichtlich des Sturzrisikos verwendet.

In einem optionalen Verfahrensschritt 66 werden die Bewegungsanalyse und das Sturzrisiko an einen Leistungserbringer des Gesundheitswesens, beispielsweise an einen Arzt oder medizinischen Fachmann, übermittelt. Dadurch ist beispielsweise eine Fern-Überwachung von ANS-Störungen des Hörsystemnutzers ermöglicht. Vorzugsweise wird dieser Verfahrensschritt 66 lediglich ausgeführt, wenn der Hörsystemnutzer eine entsprechende Zustimmung oder Freigabe erteilt hat.

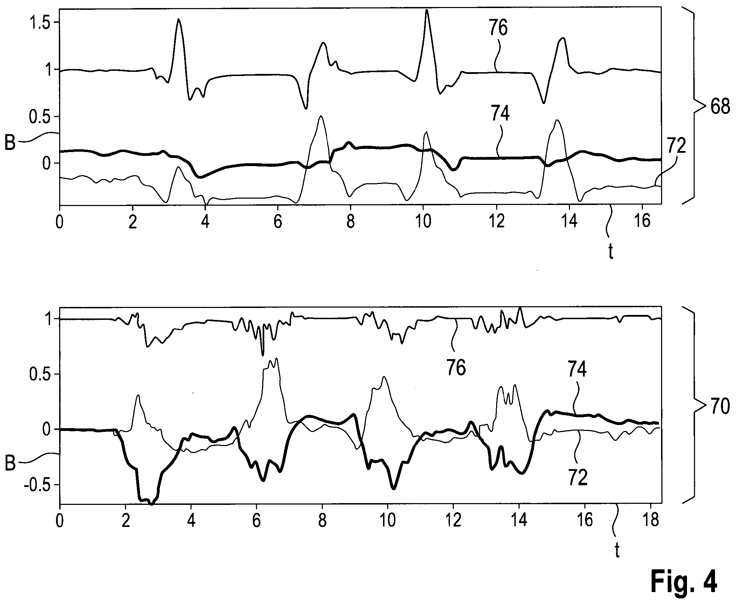

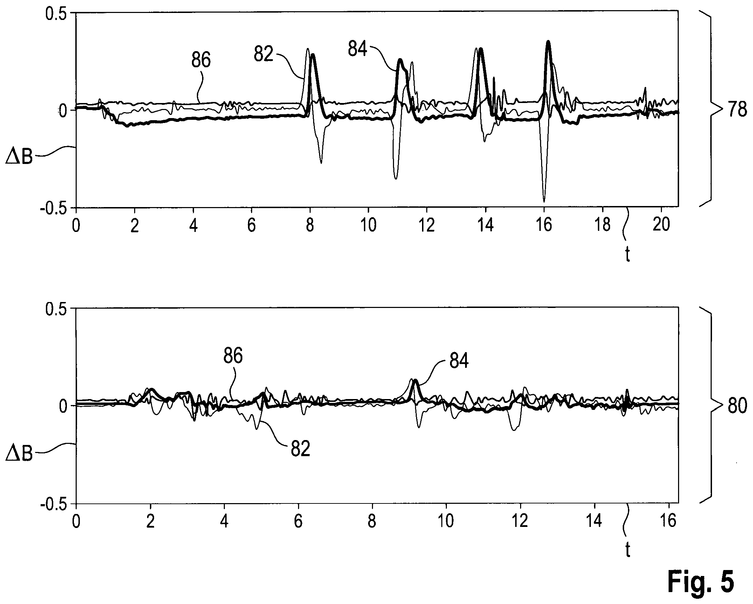

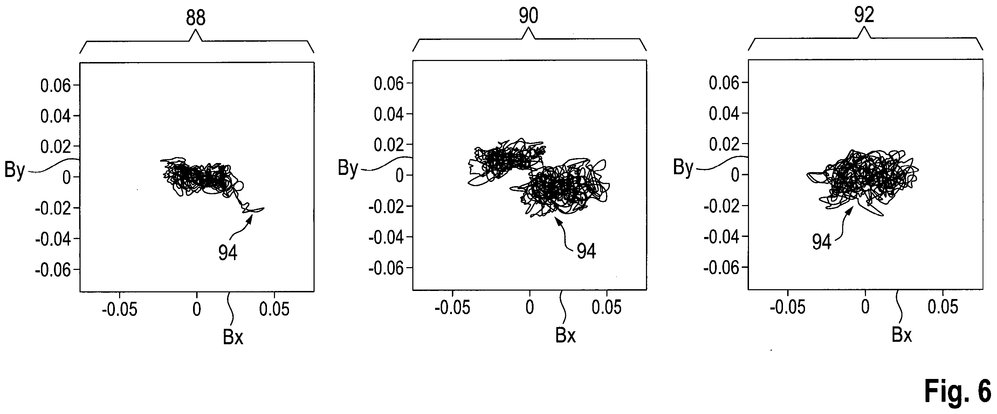

Nachfolgend sind anhand der Figuren 4 bis 6 Bewegungsdaten 26 für unterschiedliche Bewegungsereignisse gezeigt. Die Bewegungsdaten 26 sind hierbei mit einem binauralen Hörgeräte 4 erfasst, welches mit einem Bewegungssensor 24, insbesondere einem 3D-Beschleunigungssensor, ausgestattet ist. Die Figuren zeigen hierbei die Bewegungsdaten 26 für "stationären Übungen". Die Bewegungsdaten 26 der Fig. 4 zeigen ein Aufstehen von einem Stuhl, also einen Bewegungsübergang von Sitzen zu Stehen. In der Fig. 5 ist der Signal- oder Datenverlauf für einen Umdrehungstest, und in der Fig. 6 sind Bewegungsdaten 26 eines Still-Steh-Tests gezeigt.

Die Fig. 4 umfasst zwei vertikal übereinander angeordnete Abschnitte 68, 70, welche jeweils ein Zeit-Beschleunigungs-Diagramm zeigen. In den Abschnitten 68, 70 ist horizontal, also entlang der Abszissenachse (X-Achse), die Zeit t, beispielsweise in Sekunden, und entlang der vertikalen Ordinatenachse (Y-Achse) eine Beschleunigung B aufgetragen. Positive Beschleunigungswerte entsprechen einer Beschleunigung und negative Beschleunigungswerte einem Abbremsen. Die Abschnitte 68, 70 zeigen hierbei Rohmessdaten eines 3-Achsen-Beschleunigungsmessers (3D-Beschleunigungssensor), normiert auf die Erdschwere G (1 G = 9,81 m/s²).

In den Abschnitten 68, 70 sind die Bewegungsdaten 26 anhand von jeweils drei Verläufen 72, 74, 76 dargestellt, wobei der Verlauf 72 die Beschleunigung B entlang einer X-Richtung, und der Verlauf 74 die Beschleunigung B entlang einer Y-Richtung, und der Verlauf 76 die Beschleunigung B entlang einer Z-Richtung zeigt.

Die X-, Y-, und Z-Richtung bezieht sich hierbei beispielsweise auf die drei Hauptscharen der Körperebenen, also Transversalebenen (XY), Frontalebenen (YZ), und Sagittalebenen (XZ). Die Die Abszissenachse (X-Achse, X-Richtung) ist hierbei beispielsweise entlang der Sagittalrichtung (vorne, hinten) und die Ordinatenachse (Y-Achse, Y-Richtung) entlang der Transversalrichtung (links, rechts) sowie die Applikatenachse (Z-Achse, Z-Richtung) entlang der Longitudinalrichtung (oben, unten) orientiert.

Die Abschnitte 68, 70 zeigen hierbei die Bewegungsdaten 26 für zwei Situationen, wobei lediglich die monauralen Bewegungsdaten für eines der Einzelgeräte 6a, 6b, beispielsweise dem am linken Ohr getragenen Einzelgerät, dargestellt ist.

Die Verläufe 72, 74, 76 in dem Abschnitt 68 zeigen die Bewegungsdaten 26 für den Fall, dass der Hörsystemnutzers zweimal hintereinander von einem Stuhl in normaler Weise aufsteht, und sich anschließend wieder hinsetzt.

In dem Abschnitt 70 sind die gleichen Verläufe für ein zweimaliges Aufstehen und Hinsetzen gezeigt, wobei sich der Hörsystemnutzer hierbei an einer Armstütze abgestützt hat, um eine einseitige Behinderung zu simulieren.

Im Normalfall des Abschnitts 68 sind Beschleunigungen in entlang der Z- und X-Richtung sichtbar. Die X-Komponente ist auf Beschleunigungen durch die Vorwärtsneigung des Körpers zurückzuführen, während die Z-Komponente die Beschleunigungen durch das Anheben oder Bewegen des Körpers beschreibt. Im armlehnengestützten Fall des Abschnitts 70 ist die X-Komponente dem ersten Fall ähnlich. Die Z-Komponente ist jedoch viel weniger ausgeprägt. Darüber hinaus gibt es auch eine Y-Komponente, welche zeigt, dass es eine signifikante Bewegung zur Seite gab. Insgesamt dauert auch die Zeitspanne für die Sequenz für den unterstützten Fall länger (1-2 Sekunden) im Vergleich zum Normalfall (<1 Sekunde).

Durch Auswertung und Analyse der Bewegungsdaten 26 beziehungsweise der Verläufe 72, 74, 76 ist es somit möglich, verschiedene Arten des Aufstehens/Sitzens mit Hörsystemen 2, welche mit einem Beschleunigungsmesser 24 ausgestattet sind, zu quantifizieren und zu unterscheiden.

Die Fig. 5 umfasst zwei vertikal übereinander angeordnete Abschnitte 78, 80, welche jeweils ein Zeit-Beschleunigungs-Diagramm zeigen. In den Abschnitten 68, 70 ist horizontal, also entlang der Abszissenachse (X-Achse), die Zeit t, beispielsweise in Sekunden, und entlang der vertikalen Ordinatenachse (Y-Achse) eine Beschleunigungsdifferenz ΔB aufgetragen. Die Beschleunigungsdifferenz ΔB ist die Differenz zwischen den Beschleunigungen des linken und rechten Einzelgeräts 6a, 6b, also die binaurale Differenz, dies bedeutet die Beschleunigung des linken Geräts minus Beschleunigung des rechten Geräts, wobei die Beschleunigungsdifferenz ΔB auf die Erdschwere G normiert ist.

In den Abschnitten 78, 80 sind die Bewegungsdaten 26 anhand von jeweils drei Verläufen 82, 84, 86 dargestellt, wobei der Verlauf 82 die Beschleunigungsdifferenz ΔB entlang der X-Richtung, und der Verlauf 84 die Beschleunigungsdifferenz ΔB entlang der Y-Richtung, und der Verlauf 86 die Beschleunigungsdifferenz ΔB entlang der Z-Richtung zeigt.

Das Drehverhalten einer Person ist mit dem Sturzrisiko verbunden. Die Abschnitte 78, 80 zeigen hierbei die Fähigkeit, circa 90° Körperdrehungen mit Beschleunigungsmesserdaten für eine binaurale Aufstellung zu messen. Die Abschnitte 78,80 zeigen hierbei die Bewegungsdaten für die Drehungssequenz 90° links, 90° rechts, 90° links, 90° rechts.

Die Verläufe 82, 84,86 des Abschnitts 78 zeigen die Bewegungsdaten für Drehungen in normaler Geschwindigkeit und die Verläufe 82, 84, 86 des Abschnitts 80 zeigen die Bewegungsdaten für Drehungen in langsamer Geschwindigkeit.

Im Normalgeschwindigkeitsfall des Abschnitts 78 sind deutliche Beschleunigungsspitzen in der X- und Y-Komponente sichtbar, welche die tangentialen und radialen Kräfte der Körperdrehung darstellen. Im langsamen Geschwindigkeitsfall des Abschnitts 80 sind die Spitzen weniger ausgeprägt oder im Wesentlichen nicht wahrnehmbar.

Die Fig. 5 zeigt, dass Körperdrehungen mit normaler Geschwindigkeit von Körperdrehungen mit langsamer Geschwindigkeit unterschieden werden können, wenn nur ein binauraler Aufbau und Beschleunigungsmesser verwendet werden. Bei Körperdrehungen mit normaler Geschwindigkeit wird das Manöver zuverlässig und qualifiziert erkannt (Geschwindigkeit, Dauer, Drehwinkel).

Die Fig. 6 weist drei horizontale nebeneinander angeordnete Abschnitte 88, 90, 92 auf, welche jeweils ein zweidimensionales Beschleunigungs-Diagramm zeigen. In den Abschnitten 88, 90, 92 ist horizontal, also entlang der Abszissenachse (X-Achse), eine Beschleunigung Bx in X-Richtung, und entlang der vertikalen Ordinatenachse (Y-Achse) eine Beschleunigung By in Y-Richtung in Einheiten der Erdschwere G aufgetragen.

Die Abschnitte 88, 90, 92 zeigen einen Stillsteh-Test, bei welchem ein Hörsystemnutzer seinen Körper beim normalen Stehen ausbalanciert. Die Abschnitte 88, 90, 92 zeigen einen zeitlichen Verlauf 94 der Beschleunigungen für eine Messdauer von etwa einer Minute (1 min) für eine gesunde Testperson. Der Abschnitt 88 zeigt ein normales Stehen, wobei der Abschnitt 90 ein Stehen zeigt, bei welchem die Testperson die Augen geschlossen hat. Die Bewegungsdaten des Abschnitts 92 zeigen ein einbeiniges Stehen, also ein Stehen auf einem Bein, bei geöffneten Augen.

Die Abschnitte 88, 90, 92 zeigen jeweils die mittelwertfreien Beschleunigungsmesserdaten in der XY-Ebene für eine monaurale Beschleunigungsmessung. Perfekte Balancierfähigkeiten würden einen Punkt im Ursprung (0, 0) ergeben. Große Schwankungen in den Kurven sind Anzeichen für eine stärkere Notwendigkeit, das Gleichgewicht zu regulieren.

Die Bewegungsdaten des Abschnitts 88 zeigen einen am Ursprung zentrierten Cluster, mit einer flachen Gesamtform. Die Bewegungsdaten weisen größere Variationen in der X- als in der Y-Komponente auf, welches einer dominanten Links/Rechts-Fluktuation und weniger ausgeprägten Vorwärts/Rückwärts-Fluktuationen entspricht.

Die Bewegungsdaten des Abschnitts 88 zeigen zwei Cluster, welche um den Ursprung verteilt angeordnet sind. Aufgrund der geschlossenen Augen fehlt ein visueller Referenzpunkt, und das Gleichgewicht wird nur durch das Gleichgewichtssystem gehalten. Dies führt dazu, dass die Testperson in semi-stabile Zustände gerät, bis die Fluktuationen zu groß werden und sich in einem anderen semistabilen Zustand stabilisieren.

Die Bewegungsdaten des Abschnitts 90 zeigen einen Cluster nahe am Ursprung. Die Gesamtform des Clusters ist im Vergleich zum Stehen (Abschnitt 88) kreisförmiger, wobei links/rechts und vorne/hinten Fluktuationen auftreten, welche auf eine dynamischere muskuläre Regulation zum Halten des Gleichgewichts hinweisen.

Beispielsweise ist es denkbar im Rahmen des Verfahrens 40 zumindest das normale Steh-Szenario jeden Tag auszuführen, wobei Abweichungen von den typischen Ergebnissen beispielsweise einen Hinweis auf einen schlechten Tag mit einem erhöhten Sturzrisiko geben.

Die Erfindung ist nicht auf die vorstehend beschriebenen Ausführungsbeispiele beschränkt. Vielmehr können auch andere Varianten der Erfindung von dem Fachmann hieraus abgeleitet werden, ohne den Gegenstand der Erfindung zu verlassen. Insbesondere sind ferner alle im Zusammenhang mit den Ausführungsbeispielen beschriebenen Einzelmerkmale auch auf andere Weise miteinander kombinierbar, ohne den Gegenstand der Erfindung zu verlassen.

### Bezugszeichenliste

- 2: Hörsystem
- 4: Hörgerät
- 6a, 6b: Einzelgerät
- 8: Kommunikationsverbindung
- 10: Gerätegehäuse
- 12: Eingangswandler
- 14: Akustikdaten/Audiosignal
- 16: Signalverarbeitungssignal
- 18: Ausgangssignal
- 20: Ausgangswandler
- 22: Batterie
- 24: Bewegungssensor
- 26: Bewegungsdaten
- 28, 30: Transceiver
- 32: Kommunikationsverbindung
- 34: Zusatzgerät/Smartphone
- 36: Anzeigeeinheit
- 38: Anwendungssoftware
- 40: Verfahren
- 42 ... 66: Verfahrensschritt
- 68, 70: Abschnitt
- 72, 74, 76: Verlauf
- 78,80: Abschnitt
- 82, 84, 86: Verlauf
- 88, 90, 92: Abschnitt
- 94: Verlauf

- t: Zeit
- B, Bx, By: Beschleunigung
- ΔB: Beschleunigungsdifferenz

## Patentansprüche

1. Verfahren (40) zum Betreiben eines Hörsystems (2), welches ein Hörgerät (4) mit mindestens einem Eingangswandler (12) und mit einem Ausgangswandler (20) sowie mit einem Bewegungssensor (24) aufweist,
- bei welchem eine Bewegung eines Hörsystemnutzers als Bewegungsdaten (26) des Bewegungssensors (24) erfasst wird,
- bei welchem anhand der erfassten Bewegungsdaten (26) eine Wahrscheinlichkeit für ein zukünftiges Fall- oder Sturzereignis des Hörsystemnutzers bestimmt wird, wobei zur Bestimmung der Wahrscheinlichkeit einen Gang des Hörsystemnutzers charakterisierende Makro- und Mikroereignisse, Dreh- oder Rotationsverhalten des Hörsystemnutzers, oder Übergänge zwischen Bewegungen und/oder Kombinationen von Bewegungen ausgewertet werden,
- bei welchem ein wahrnehmbares Warnsignal erzeugt wird, wenn die Wahrscheinlichkeit einen hinterlegten Schwellwert erreicht oder überschreitet,
**dadurch gekennzeichnet, dass**
- die Einstellungen des Hörgeräts (4) hinsichtlich einer Signalverarbeitung und Ausgabe eines Umgebungsschalls bei einer Änderung der Wahrscheinlichkeit derart variiert werden, dass ein Sturzrisiko minimiert wird.

2. Verfahren (40) nach Anspruch 1,
**dadurch gekennzeichnet,**
**dass** in einem Speicher des Hörsystems (2) persönliche Gesundheitsdaten des Hörsystemnutzers hinterlegt werden, und dass die Bestimmung der Wahrscheinlichkeit anhand der hinterlegten Gesundheitsdaten erfolgt.

3. Verfahren (40) nach Anspruch 1 oder 2,
**dadurch gekennzeichnet,**
- **dass** anhand von Akustikdaten (14) des Eingangswandlers (12) eine Umgebungssituation des Hörsystemnutzers bestimmt wird, und
**dass** die Bewegungsdaten (26) der Umgebungssituation zugeordnet und in einem Speicher hinterlegt werden.

4. Verfahren (40) nach Anspruch 3,
**dadurch gekennzeichnet,**
**dass** für die Bestimmung der Wahrscheinlichkeit die Bewegungsdaten (26) für eine jeweilige Umgebungssituation mit den hinterlegten Bewegungsdaten und Umgebungssituationen verglichen werden.

5. Verfahren (40) nach Anspruch 3 oder 4,
**dadurch gekennzeichnet,**
**dass** anhand der hinterlegten Bewegungsdaten und Umgebungssituationen ein Benutzerprofil für den Hörsystemnutzer erstellt wird.

6. Verfahren (40) nach einem der Ansprüche 1 bis 5, wobei das Hörgerät (4) binaural ausgebildet ist und hierzu zwei Einzelgeräte (6a, 6b) aufweist, und wobei jedes der Einzelgeräte (6a, 6b) einen Bewegungssensor (24) aufweist,
**dadurch gekennzeichnet,**
**dass** die Bewegungsdaten (26) der Einzelgeräte (6a, 6b) getrennt voneinander ausgewertet, und anschließend zur Bestimmung der Wahrscheinlichkeit kombiniert werden.

7. Verfahren nach einem der Ansprüche 1 bis 6,
**dadurch gekennzeichnet,**
**dass** die Bewegungsdaten (26) und/oder die Wahrscheinlichkeit an ein signaltechnisch mit dem Hörgerät (4) gekoppeltes Zusatzgerät (34) übermittelt werden.

8. Verfahren nach Anspruch 7, wobei das Zusatzgerät (34) ein mobiles Anzeige- und Bediengerät ist,
**dadurch gekennzeichnet,**
**dass** bei einem Erreichen oder Überschreiten des Schwellwerts auf dem Zusatzgerät (34) Maßnahmen zur Reduzierung eines Sturz- oder Fallrisikos angezeigt werden.

9. Verfahren nach einem der Ansprüche 1 bis 8,
**dadurch gekennzeichnet,**
**dass** eine Bewegungsanalyse durch den Hörsystemnutzer ausgelöst wird, wobei die Bewegungsdaten (26) zur Bestimmung der Wahrscheinlichkeit während eines vorgegebenen Bewegungsmusters des Hörsystemnutzers erfasst werden.

10. Hörsystem (2), insbesondere Hörhilfevorrichtung, aufweisend ein Hörgerät (4) mit mindestens einem Eingangswandler (12) zum Empfangen eines akustischen Umgebungssignals, und mit einem Ausgangswandler (20) zur Ausgabe eines akustischen Signals, sowie mit einem Bewegungssensor (24) zum Erfassen einer Bewegung eines Hörsystemnutzers und mit einem Controller zur Durchführung eines Verfahrens nach einem der Ansprüche 1 bis 9.

## Claims

1. Method (40) for operating a hearing system (2) which has a hearing device (4) with at least one input transducer (12) and with an output transducer (20) and with a motion sensor (24),
- in which a movement of a hearing system user is captured as motion data (26) from the motion sensor (24),
- in which a probability of a future fall event involving the hearing system user is determined on the basis of the captured motion data (26), wherein, in order to determine the probability, macro-events and micro-events characterizing a gait of the hearing system user, turning or rotating behaviours of the hearing system user, or transitions between movements and/or combinations of movements are evaluated,
- in which a perceptible warning signal is generated if the probability reaches or exceeds a stored threshold value,
**characterized in that**
- the settings of the hearing device (4) with regard to signal processing and output of an ambient sound are varied, if the probability changes, in such a manner that a fall risk is minimized.

2. Method (40) according to Claim 1, **characterized in that** personal health data relating to the hearing system user are stored in a memory of the hearing system (2), and **in that** the probability is determined on the basis of the stored health data.

3. Method (40) according to Claim 1 or 2, **characterized**
- **in that** an environmental situation of the hearing system user is determined on the basis of acoustic data (14) from the input transducer (12), and
**in that** the motion data (26) are assigned to the environmental situation and are stored in a memory.

4. Method (40) according to Claim 3, **characterized in that**, in order to determine the probability, the motion data (26) for a respective environmental situation are compared with the stored motion data and environmental situations.

5. Method (40) according to Claim 3 or 4, **characterized in that** a user profile for the hearing system user is created on the basis of the stored motion data and environmental situations.

6. Method (40) according to one of Claims 1 to 5, wherein the hearing device (4) is of binaural design and for this purpose has two individual devices (6a, 6b), and wherein each of the individual devices (6a, 6b) has a motion sensor (24), **characterized in that** the motion data (26) relating to the individual devices (6a, 6b) are evaluated separately from one another and are then combined in order to determine the probability.

7. Method according to one of Claims 1 to 6, **characterized in that** the motion data (26) and/or the probability is/are transmitted to an additional device (34) coupled to the hearing device (4) using signalling.

8. Method according to Claim 7, wherein the additional device (34) is a mobile display and operating device, **characterized in that** measures for reducing a fall risk are displayed on the additional device (34) when the threshold value is reached or exceeded.

9. Method according to one of Claims 1 to 8, **characterized in that** a motion analysis is initiated by the hearing system user, wherein the motion data (26) for determining the probability are captured during a predefined motion pattern of the hearing system user.

10. Hearing system (2), in particular hearing aid apparatus, having a hearing device (4) with at least one input transducer (12) for receiving an acoustic environmental signal and with an output transducer (20) for outputting an acoustic signal and with a motion sensor (24) for capturing a movement of a hearing system user and with a controller for carrying out a method according to one of Claims 1 to 9.

## Revendications

1. Procédé (40) de fonctionnement d'un système auditif (2) qui comprend une prothèse auditive (4) dotée d'au moins un transducteur d'entrée (12) et d'un transducteur de sortie (20) ainsi que d'un capteur de mouvement (24),
- dans lequel un mouvement d'un utilisateur du système auditif est détecté en tant que données de mouvement (26) du capteur de mouvement (24),
- dans lequel une probabilité pour un futur événement de chute de l'utilisateur du système auditif est déterminée sur la base des données de mouvement détectées (26), dans lequel des macro et micro-événements caractérisant une démarche de l'utilisateur du système auditif, le comportement de pivotement ou de rotation de l'utilisateur du système auditif, ou des transitions entre des mouvements et/ou des combinaisons de mouvements sont évalués pour déterminer ladite probabilité,
- dans lequel un signal d'avertissement perceptible est généré lorsque la probabilité atteint ou dépasse une valeur de seuil stockée, **caractérisé en ce que**
- les réglages de la prothèse auditive (4) en ce qui concerne le traitement du signal et la sortie d'un son ambiant en cas de modification de la probabilité sont modifiés de manière à minimiser le risque de chute.

2. Procédé (40) selon la revendication 1,
caractérisé
en que des données de santé personnelles de l'utilisateur du système auditif sont stockées dans une mémoire du système auditif (2), et en ce que la probabilité est déterminée sur la base des données de santé stockées.

3. Procédé (40) selon la revendication 1 ou 2,
**caractérisé**
- **en ce qu'**une situation ambiante de l'utilisateur du système auditif est déterminée sur la base de données acoustiques (14) du transducteur d'entrée (12), et
**en ce que** les données de mouvement (26) sont associées à la situation ambiante et stockées dans une mémoire.

4. Procédé (40) selon la revendication 3,
**caractérisé**
**en ce que**, pour déterminer ladite probabilité, les données de mouvement (26) sont comparées aux données de mouvement et aux situations ambiantes stockées pour une situation ambiante respective.

5. Procédé (40) selon la revendication 3 ou 4,
**caractérisé**
**en ce qu'**un profil d'utilisateur est créé sur la base des données de mouvement stockées et des situations ambiantes pour l'utilisateur du système auditif.

6. Procédé (40) selon l'une quelconque des revendications 1 à 5, dans lequel la prothèse auditive (4) est réalisée de manière binaurale et présente à cet effet deux dispositifs individuels (6a, 6b), et dans lequel chacun des dispositifs individuels (6a, 6b) comporte un capteur de mouvement (24),
**caractérisé**
**en ce que** les données de mouvement (26) des dispositifs individuels (6a, 6b) sont évaluées séparément les unes des autres puis combinées pour déterminer ladite probabilité.

7. Procédé selon l'une quelconque des revendications 1 à 6,
**caractérisé**
**en ce que** les données de mouvement (26) et/ou la probabilité sont transmises à un dispositif auxiliaire (34) qui est couplé à la prothèse auditive (4) par une technique d'échange de signaux.

8. Procédé selon la revendication 7, dans lequel le dispositif auxiliaire (34) est un dispositif d'affichage et de commande mobile,
**caractérisé**
**en ce que**, lorsque la valeur de seuil est atteinte ou dépassée, des mesures destinées à réduire le risque de chute sont affichées sur le dispositif auxiliaire (34).

9. Procédé selon l'une quelconque des revendications 1 à 8,
**caractérisé**
**en ce qu'**une analyse de mouvement est déclenchée par l'utilisateur du système auditif, les données de mouvement (26) étant détectées pendant un mouvement type prédéfini de l'utilisateur du système auditif pour déterminer ladite probabilité.

10. Système auditif (2), en particulier appareil auditif, comprenant une prothèse auditive (4) dotée d'au moins un transducteur d'entrée (12) destiné à recevoir un signal acoustique ambiant, d'un transducteur de sortie (20) destiné à délivrer un signal acoustique, d'un capteur de mouvement (24) destiné à détecter un mouvement d'un utilisateur d'un système auditif, et d'un dispositif de commande destiné à mettre en œuvre un procédé selon l'une quelconque des revendications 1 à 9.
